# EUROPEAN PATENT APPLICATION

(11) **EP 4 112 640 A1**
(43) Date of publication of application: **04.01.2023**
(21) Application number: 21182579.9
(22) Date of filing: 29.06.2021
(51) Int. Cl.: C07K 16/28

(54) **NOVEL SINGLE DOMAIN ANTIGEN BINDING MOLECULES AND THEIR USES**

(71) Applicant: NMI Naturwissenschaftliches und Medizinisches Institut an der Universität Tübingen, 72770 Reutlingen (DE); Eberhard Karls Universität Tübingen, 72074 Tübingen (DE)
(72) Inventor: Kaiser, Philipp, 72076 Tübingen (DE); Tränkle, Björn, 72770 Reutlingen (DE); Rothbauer, Ulrich, 72072 Tübingen (DE); Sonanini, Dominik, 72074 Tübingen (DE); Kneilling, Manfred, 85298 Scheyern (DE); Pichler, Bernd, 85298 Fernhag (DE); Beschorner, Niklas, 20255 Hamburg (DE); Hamann, Martin, 22089 Hamburg (DE); Hauber, Joachim, 67061 Ludwigshafen am Rhein (DE)
(74) Representative: Witte, Weller & Partner Patentanwälte mbB

(57) **Abstract**

The present invention relates to novel single domain antibodies/nanobodies against human CD4, as well as their diagnostic/prophylactic, or therapeutic use or appliance. The nanobodies of the invention may be associated, e.g., with detectable labels, viral particles, or therapeutic moieties.

## Description

The present invention relates to novel single-domain-antibodies (sdAb), which are also known as nanobodies, which bind to human CD4, as well as to the use of such nanobodies, in particular for prophylactic, therapeutic, diagnostic or cell-targeting purposes.

The present invention also relates to polypeptides comprising or essentially consisting of one or more of such nanobodies. The invention also relates to nucleic acids encoding such nanobodies and polypeptides, to compositions comprising such nanobodies or polypeptides, and to uses of such polypeptides, nucleic acids, and compositions, in particular for prophylactic, therapeutic, diagnostic or cell-targeting purposes.

### BACKGROUND

Presently, there is an increasing importance of immunotherapies for the treatment of infections, immune-mediated inflammatory diseases (IMIDs), autoimmune diseases and cancer; thus, novel and reliable probes to detect and monitor the distribution and infiltration of different immune cell populations are urgently needed.

In precision medicine, diagnostic classification of disease-associated immune status should guide the selection of appropriate therapies. A comprehensive analysis of a patient's specific immune cell composition, activation state, and infiltration of affected tissue has been shown to be highly informative for patient stratification. When administering immunotherapeutics, this enables prediction of responders and non-responders, monitoring of therapeutic efficacy, and detection of serious adverse events even before symptoms occur.

CD4+ T cells are a key determinant of immune status due to their essential role in orchestrating immune responses in T cell-mediated delayed hypersensitivity reactions in autoimmune diseases, inflammation, cancer, and chronic viral infections.

CD4 (cluster of differentiation 4) is a glycoprotein found on the surface of immune cells such as T helper cells, monocytes, macrophages, and dendritic cells. CD4+ T helper cells are white blood cells that are an essential part of the human immune system. They are often referred to as CD4+ cells, T-helper cells or T4 cells; their main roles is to send signals to other types of immune cells, including CD8 killer cells, which then destroy the infectious particle.

CD4 is a monomeric type I transmembrane glycoprotein consisting of four N-terminal N-Immunoglobulin (Ig)-like extracellular domains connected by a short stalk to a C-terminal transmembrane domain and a cytoplasmic tail domain. Domains D1 and D3 resemble Ig variable domain and domains D2 and D4 resemble the Ig constant domain. The domain D1 interacts with class II MHC molecules. CD4 helps T-cell receptors (TCR) interact with antigen-presenting cells. The cytoplasmic C-terminal tail of CD4 interacts with tyrosine kinase Lck which activates molecular components of the signaling pathway.

CD4 binds MHC class II with exceptionally low affinity compared to all other leukocyte cell-cell recognition molecules characterized to date.

Detailed monitoring of the dynamic distribution of CD4+ T cells, accordingly, is highly relevant for companion diagnostics. Currently, the presence, activation and differentiation state of CD4+ T cells can be assessed and monitored in the peripheral blood of patients. More comprehensive diagnostic measures from biopsies of tumors or of immune-mediated inflammatory diseases (IMIDs) are enabled by a range of methods including intra-cytoplasmic flow cytometry analysis (IC-FACS), cytometry by time of flight (CyTOF), immunohistochemistry and *ex vivo* cytokine assays or RT-PCR analysis. Due to the heterogeneity of cancers and the inhomogeneity of the CD4+ T cell infiltrate in IMIDs, biopsies provide highly variable information about a very small area and thus are disqualified to estimate the total CD4+ T cell infiltrate.

Considering the emerging role of infiltrating lymphocytes and the impact of CD4+ T cells on the outcome of immunotherapies novel holistic approaches are needed to assess CD4+ T cells, and other CD4 expressing cells, throughout the entire tumor, all metastasis, or the affected tissue of IMIDs.

In view of the above, it is an object of the present invention to provide novel tools for identifying or targeting CD4+ cells, as well as to enable a therapeutic, diagnostic or prophylactic use of tools identifying or targeting CD4+ cells.

### SUMMARY

The present invention provides a nanobody (or single-domain-antibody) which binds to human CD4, the nanobody comprising
a) the amino acid sequences (i) SGFTFSKL (SEQ ID NO: 1) as CDR1, (ii) IDSSGDTTDYLA (SEQ ID NO: 2) as CDR2 and (iii) REDPPG (SEQ ID NO: 3) as CDR3; or
b) the amino acid sequences (i) SGFDVDYY (SEQ ID NO: 4) as CDR1, (ii) IASSDGSTYYAD (SEQ ID NO: 5) as CDR2 and (iii) DATCPYYCSGSVCYLETGMD (SEQ ID NO: 6) as CDR3; or
c) the amino acid sequences (i) SGFALEYY (SEQ ID NO: 7) as CDR1, (ii) MSASGGVINYSE (SEQ ID NO: 8) as CDR2 and (iii) EKAYYGSSWAECYLMMD (SEQ ID NO: 9) as CDR3; or
d) amino acid sequences that have at least 90% sequence homology with the amino acid sequences as defined in a), b) or c),
or a functionally conservative variant of the nanobody as defined in any of a), b) or c) comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9.

The novel single domain antibodies (or nanobodies) of the invention specifically recognize the human CD4 receptor in its native state on various CD4+ cells. With the novel nanobodies disclosed herein, it is possible to selectively identify or target CD4+ cells, such as CD4+ T cells, which makes them a valuable tool in diagnosis, prophylaxis and therapy, alone or in combination/association with additional substances/molecules or methods.

A single domain antibody (sdAb) is an antibody fragment consisting of a single monomeric variable antibody domain, which is able to selectively bind to a specific antigen. As single domain antibodies the nanobodies of the invention represent an excellent alternative to conventional antibodies (IgGs): Antibodies from camelids, such as llamas, include a unique subset of immunoglobulins consisting of heavy chain homodimers devoid of light chains. Their variable region (V_{H}H) is the smallest antigen-binding fragment found in the antibody world, and as a single polypeptide chain it is especially suitable for protein engineering. Single domain antibodies, or "nanobodies", are the recombinant minimal-sized, intact antigen-binding domains derived from the V_{H}H region of these heavy-chain antibodies. Unlike monoclonal antibodies, they can be readily produced in large amounts in simple bacterial expression systems. Moreover, nanobodies are usually extremely stable, can bind antigens with affinities in the nanomolar range, and are smaller in size (approximately 15 kDa) and thereby easier to manipulate genetically as compared with antibody fragments such as single chain variable fragments (ScFvs) comprising the variable domains of heavy and light chains of a conventional IgG.

Also, due to the small size and compact folding nanobodies show a high chemical stability, solubility and fast tissue penetration. Additionally, nanobodies can be easily converted into multivalent formats, such as biparatopic, bivalent, trivalent, tetravalent or other multi-specific constructs, e.g. addressing different epitopes on the same antigen, which the nanobodies presented herein can also be used for according to the invention. Also, nanobodies have comparable antigen specificities and affinities and, due to their high homology with human antibody (VH) fragments, show only very low immunogenicity.

In a preferred embodiment, the nanobody of the invention binds to a region of a domain of human CD4, which region is distinct from the gp120 binding site to CD4. gp120 or the "envelope glycoprotein GP120" is a glycoprotein exposed on the surface of the HIV (human immunodeficiency virus) envelope, binding to CD4+ cells.

According to one embodiment, the nanobody of the invention binds to domain D1 or to domain D3 of human CD4.

Also, in a preferred embodiment, the nanobody of the invention is selected from a nanobody which specifically binds to domain D1 of human CD4, wherein preferably, the nanobody comprises the amino acid sequences (i) SGFTFSKL (SEQ ID NO: 1) as CDR1, (ii) IDSSGDTTDYLA (SEQ ID NO: 2) as CDR2 and (iii) REDPPG (SEQ ID NO: 3) as CDR3; or the amino acid sequences (i) SGFALEYY (SEQ ID NO: 7) as CDR1, (ii) MSASGGVINYSE (SEQ ID NO: 8) as CDR2 and (iii) EKAYYGSSWAECYLMMD (SEQ ID NO: 9) as CDR3; or a functionally conservative variant of these nanobodies comprising a conservative substitution of one or two amino acids in one, two or three of the sequences, respectively, SEQ ID No. 1, SEQ ID No. 2, and SEQ ID No. 3; or SEQ ID No. 7, SEQ ID No. 8, and SEQ ID No. 9.

The above nanobodies, while binding to the same domain of CD4, i.e. domain D1, however, bind to different epitopes of CD4.

In one embodiment, the nanobody binds to domain D3 of CD4, and comprises the amino acid sequences (i) SGFDVDYY (SEQ ID NO: 4) as CDR1, (ii) IASSDGSTYYAD (SEQ ID NO: 5) as CDR2 and (iii) DATCPYYCSGSVCYLETGMD (SEQ ID NO: 6) as CDR3; or amino acid sequences that have at least 90% sequence homology with the above amino acid sequences, or a functionally conservative variant of the nanobody as defined above comprising a conservative substitution of one or two amino acids in one, two or three of the sequences, respectively, SEQ ID No. 4, SEQ ID No. 5, or SEQ ID No. 6.

Domain D1 of CD4 is 100 amino acids long, and designated to comprise amino acid positions 26 to 125, whereas domain D3 is 114 amino acids long and designated to comprise amino acid positions 204 to 317.

According to a preferred embodiment, the nanobody of the invention binds to domain D1 of human CD4 between amino acid Thr 17 and Lys 75, or to domain 1 of human CD4 between amino acid Thr 17 and Glu 91, and, accordingly, the nanobody of the invention is also a nanobody binding in the respective regions and comprising an amino acid structure as defined herein.

Further, according to a preferred embodiment, the nanobody of the invention comprises four framework regions (FR1 to FR4) and three complementarity determining regions (CDR1 to CDR3), the three complementarity determining regions consisting of
(i) one of the amino acid sequences a) to c), or of
(ii) an amino acid sequence that has at least 90% sequence homology with one of the amino acid sequences a) to c), or of
(iii) an amino acid sequence comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9;
wherein preferably, the nanobody comprises an amino acid sequence selected from the group consisting of the amino acid sequences SEQ ID NO. 10, SEQ ID NO. 11, SEQ ID NO. 12, or an amino acid sequence that has at least 90% sequence homology with one of the amino acid sequences SEQ ID NO. 10, SEQ ID NO. 11, SEQ ID NO. 12.

Within the present invention, it has been shown that the nanobodies of the invention do not affect T cell proliferation or cytokine expression *in vitro.*

Also, by labeling the nanobodies of the invention, it was/is possible to track the presence and location of CD4+ cells in, e.g., murine xenograft models.

Using the nanobodies of the invention, it was also possible to demonstrate a high signal-to-noise ratio in mouse optical imaging.

As used herein, the articles "a" and "an" refer to one or to more than one (e.g., to at least one) of the grammatical object of the article.

The term "or" is used herein to mean, and is used interchangeably with, the term "and/or", unless context clearly indicates otherwise.

The terms "proteins" and "polypeptides" are used interchangeably herein, as well as the terms "nanobody" and "single domain antibody".

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

The methods and compositions of the present invention encompass polypeptides and nucleic acids having the sequences specified, or sequences substantially identical or similar thereto, e.g., sequences at least 90%, 91%, 92%, 93%, 94%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences containing a common structural domain having at least about 90%, 91%, 92%, 93%, 94%, 95%, 96%, 97%, 98% or 99% identity to a reference sequence. In other embodiments, the amino acid sequence can contain one or more amino acid insertions, deletions, or substitutions (e.g., conservative substitutions) to arrive at a percentage identity of at least about 90%, 91 %, 92%, 93%, 94%, 95%, 96%, 97%, 98% or 99% identity to a reference sequence. In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 80%, 85%, 90%, 91%, 92%, 93%, 94%, 95%, 96%, 97%, 98% or 99% identity to a reference sequence.

A "functionally conservative variant" of the nanobody of the invention as used herein means a nanobody or fragment in which one or more amino acid residues have been modified without altering desired properties, such as antigen affinity and/or antigen specificity. Such variants include, but are not limited to, certain amino acids substituted with amino acids having similar properties. E.g., a "conservative amino acid substitution" is one in which the amino acid residue is replaced with an amino acid residue having a similar side chain. Families of amino acid residues having similar side chains have been defined in the art. Such conservative substituents are preferably substituted by another amino acid residue of the following groups (a) to (e): (a) a small aliphatic, nonpolar or weakly polar residue (Ala, Ser Thr, Pro, Gly), (b) polar, negatively charged residues and their (uncharged) amides (Asp, Asn, Glu, Gln), (c) polar, positively charged residues (His, Arg, Lys) ), (D) large aliphatic, nonpolar residues (Met, Leu, Ile, Val, Cys), and (e) aromatic residues (Phe, Tyr, Trp).

Particularly preferred conservative substitutions are: Ala to Gly or Ser, Arg to Lys, Asn to Gln or His, Asp to Glu, Cys to Ser, Gln to Asn, Glu to Asp, Gly to Ala or Pro, His To Asn or Gln, Ile to Leu or Val, Leu to Ile or Val, Lys to Arg or Gln or Glu, Met to Leu or Tyr or Ile, Phe to Met or Leu or Tyr, Ser to Thr, Thr to Ser, Trp To Tyr, Tyr to Trp, and / or Phe to Val or Ile or Leu.

The amino acid sequence and structure of nanobodies can be considered to include, but are not limited to, four framework regions or "FRs", which are interrupted by three complementarity determining regions or "CDRs". The total number of amino acid residues in the nanobody may be in the range of 110-130.

Thus, according to one aspect of the invention, CD4-specific nanobodies are used for the detection and *in vivo* imaging of CD4+ cells, preferably CD4+ T cells, which makes them not only versatile probes, e.g., for immuno-PET for patient stratification and for monitoring individual immune responses during personalized immunotherapy, but also valuable tools for therapeutic or pharmaceutical uses, e.g. for cell-targeting in gene therapy.

According to one aspect of the invention, the nanobody of the invention is associated with at least one of a detectable label, a viral particle, and a therapeutically or pharmacologically active agent.

The term "associated with" when used in connection with the nanobody of the invention refers to any modification of the nanobody with another moiety that is/can be used as a diagnostic moiety/agent, or as a therapeutically or pharmacologically active moiety or agent. With the modification, the agent or moiety (the nanobody gets associated with) is indirectly or directly bound to the nanobody, or incorporated into the nanobody.

For example, such modification may comprise the introduction (e.g. by conjugation, biologically or chemically linking, covalent binding or in any other suitable manner) of one or more functional groups or agents or moieties, which can be a detectable label, a viral particle, and a therapeutically or pharmacologically active agent, and/or that, e.g., alters/increases the half-life, the solubility and/or the absorption of the nanobody, that reduce the immunogenicity and/or the toxicity of the nanobody, that eliminate or attenuate any undesirable side effects of the nanobody, and/or that confer other advantageous properties to and/or reduce the undesired properties of the nanobody; or any combination of two or more of the foregoing. Examples of such functional groups and of techniques for introducing them will be clear to the skilled person, and can generally comprise all functional groups and techniques mentioned in the general background art cited hereinabove as well as the functional groups and techniques known *per se* for the modification of pharmaceutical proteins, and in particular for the modification of antibodies or antibody fragments. Such functional groups may for example be linked directly (for example covalently) to a nanobody used in the invention, or optionally via a suitable linker or spacer, as will again be clear to the skilled person.

As used herein the terms "label", "marker" and variants thereof when used in the context of a nanobody/construct refer to a detectable compound, composition or molecule that is linked, associated or conjugated directly or indirectly to the nanobody or construct disclosed herein, to facilitate detection of the second molecule. Non-limiting examples of labels as known in the art include fluorescent tags, enzymatic linkages, and radioactive isotopes. In one example, a "labeled nanobody" refers to the direct or indirect conjugation of the detectable compound, composition or molecule to the nanobody. Additionally, or alternatively, the detectable compound, composition or molecule can be incorporated into the nanobody structure by means other than direct or indirect conjugation. An exemplary method of such incorporation is the replacement of an amino acid residue of the nanobody with a modified amino acid residue such that it becomes detectable (e.g., by radiolabeling). The label may be directly detectable or may be detectable only after contact with further compounds compositions or molecules. In one, non-limiting example, the label may be the incorporation of a radiolabeled amino acid, which is directly detectable according to methods known in the art. In additional or alternative non-limiting examples, the label may be the attachment of biotinyl moieties to the nanobody, which are detectable following contact with marked avidin (for example, streptavidin containing a fluorescent marker or enzymatic activity that can be detected by optical or colorimetric methods as known in the art). Various methods of labeling proteins are known in the art and may be used. Examples of labels for polypeptides include, but are not limited to, the following: radioisotopes or radionuklids (such as ³⁵S, ¹¹C, ¹³N, ¹⁵O, ¹⁸F, ¹⁹F, ⁹⁹TC, ¹³¹I, ³H, ¹⁴C, ¹⁵N, ⁹⁰Y, ⁹⁹Tc, ¹¹¹In and ¹²⁵I), fluorescent labels (such as fluorescein isothiocyanate (FITC), rhodamine, lanthanide phosphors), enzymatic labels (such as horseradish peroxidase, beta-galactosidase, luciferase, alkaline phosphatase), chemiluminescent markers, biotinyl groups, predetermined polypeptide epitopes recognized by a secondary reporter (such as a leucine zipper pair sequences, binding sites for secondary antibodies, metal binding domains, epitope tags), or magnetic agents (such as gadolinium chelates). In some embodiments, labels are attached by spacer arms of various lengths to reduce potential steric hindrance for the binding of the nanobody or the construct disclosed herein.

In order to label a nanobody of the invention, any method known in the field can be applied. Generally, in order to label the nanobody of the invention for, e.g, PET, PET tracers are coupled to a chelator (e.g. NODAGA, DBCO) via a reactive unit (e.g. primary amino group, azide group, C-terminal cysteine, His6-tag) located within the nanobody, via which the radionuclide is then introduced.

A "therapeutically or pharmacologically active agent" may comprise any therapeutically active agent that effects a therapeutic, i.e. beneficial treatment effect in a patient in need of a therapeutic treatment. Non-limiting examples of agents include, for example, a cytokine inhibitor, a growth factor inhibitor, an immunosuppressant, an anti-inflammatory agent, a metabolic inhibitor, an enzyme inhibitor, a cytotoxic agent, and a cytostatic agent.

According to one aspect of the invention, the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuklids or magnetic particles.

With the nanobody of the invention modified with a detectable label or "a contrast agent", different approaches for imaging cells can be performed.

Noninvasive imaging approaches offer a significant benefit compared to current diagnostic standard. To date, radiolabeled antibodies have been applied to image CD4+ T cells in preclinical models. Due to the recycling effect mediated by the neonatal Fc receptor, full-length antibodies have a long serum half-life (~ 1-3 weeks), which requires long clearance times (several days) before high-contrast images can be acquired. Additionally, effector function via the Fc region was shown to induce depletion or functional changes in CD4+ cells including the induction of proliferation or cytokine release. With the CD4-nanobodies presented herein, all these drawbacks and disadvantages can be avoided.

According to another aspect of the invention, the nanobody is associated with a viral particle, wherein the viral particle is an Adenovirus-associated Virus (AAV) particle, an Adenovirus particle, or an lentiviral particle, and wherein preferably the AAV particle is selected from an AAV2, AAV3, AAV4, AAV5, AAV6, AAV7, AAV8, or an AAV9 particle.

Accordingly, in another preferred embodiment, the nanobody of the invention is associated with a therapeutically active agent, wherein the therapeutically active agent is an active gene transfer agent.

Within the present invention it has been shown that the nanobody of the invention associated with a viral particle as indicated above can be efficiently used for targeting human CD4+ T cells, and can be used, e.g., for gene therapy, preferably for gene therapy of disease-related peripheral blood CD4+ leukocytes.

A gene transfer agent allows clinicians to introduce a therapeutic relevant nucleic acid sequence (i.e. DNA or RNA) of interest directly into a patient (*in vivo* gene therapy) or into cells isolated from a patient or a donor (*ex vivo* gene therapy). Therapeutic transgenes (encoding polypeptides, including nanobodies), produced by transduced cells after gene therapy can be maintained at a relatively constant level in a subject, as compared to a protein that is administered directly. Expression can be transient (on the order of hours to weeks) or sustained (weeks to months or longer), depending upon the specific construct used and the target tissue or cell type. These transgenes can be introduced as an integrating or non-integrating linear construct, a circular plasmid, a viral vector. Moreover, these transgenes can be introduced by virus-like particles (VLP) or chemical nanoparticle carriers (e.g. lipid nanoparticles; LNP).

The invention also concerns a nucleic acid comprising or consisting of a nucleic acid sequence coding for the nanobody of the invention, optionally linked to another nucleic sequence.

The present invention also relates to a nucleic acid encoding an amino acid sequence comprising framework region 1, CDR1, framework region 2, CDR2, framework region 3, CDR3 and framework region 4, of the nanobody as described herein, as well as a polypeptide comprising at least one nanobody as described herein.

In some of the aspects described herein, a nucleic acid sequence encoding a nanobody of the invention, or any module thereof, is operably linked to a vector. In general, as used herein, the term "vector" refers to any genetic element, such as a plasmid, phage, transposon, cosmid, chromosome, virus, virion, etc., that is capable of replication when associated with the proper control elements and that can transfer gene sequences to cells. Thus, the term includes cloning and expression vehicles, as well as viral vectors. By "recombinant vector" is meant a vector that includes a heterologous nucleic acid sequence, or "transgene", that is capable of expression *in vivo.* Vectors useful for the delivery of a sequence encoding a nanobody of the invention or components thereof can include one or more regulatory elements (e.g., promoter, enhancer, etc.) sufficient for expression of the nanobody or component thereof in the desired target cell or tissue.

In one embodiment, the nanobody of the invention is for use in diagnostic or prognostic methods, or for use as medicament, preferably for modulating CD4+ T cell function and/or cell depletion, or for use as contrast agent in non-invasive medical imaging *in vivo.*

By "diagnostic method" or "diagnosis", is meant here a method giving the possibility of determining whether an individual suffers from a pathology or certain status, e.g. treatment status, or disease status.

By "prognostic method" or "prognosis", is meant here a method giving the possibility of determining whether an individual risks developing a pathology.

Preferably, and in one embodiment of the invention, the nanobody as defined above is used for diagnosis of the infiltration of CD4+ T cells in different biological tissues or fluids.

By "use as a medicament" or drug, the general use of the nanobody as medicament or drug is meant, i.e. for treating a disease. The use can comprise a treatment method comprising the administration of a therapeutically effective amount of the nanobody or the construct of the invention to a patient in need thereof.

The diseases or conditions that may be therapeutically treated and/or monitored using the nanobody of the invention are selected from cancer, viral infections, autoimmune diseases, rheumatoid arthritis, colitis, allogenic stem cells transplantation, organ transplant rejection, acquired immunodeficiency disease.

E.g. the nanobody or the construct of the invention may be used to bock CD4 function, or its binding to MHCII, which can be used, e.g. in the treatment of cancer.

As described in greater detail below, the nanobody of the invention may for example be administered orally, via inhalation, via a parenteral route in a suitable form. When the parenteral route is contemplated, the nanobody may be in the form of injectable solutes and suspensions packaged in ampoules or flasks. The forms for parenteral administration are conventionally obtained by mixing the nanobody with buffers, stabilizers, preservatives, solubilizing agents, isotonic agents and suspension agents. According to known techniques, these mixtures are then sterilized and then packaged in the form of intravenous injections. The administered amount of nanobody or the construct naturally depends on the administration method, on the size and/or on the weight of the patient, and on the nature of the therapeutically active agent which may be associated therewith.

In one embodiment of the invention, the nanobody is used for the *in vitro* detection of CD4 in a sample.

In one embodiment, the nanobody of the invention, or the nucleic acid of the invention, is for use in retargeting AAV, Adenovirus or lentiviral vectors, or for retargeting gene transfer vectors, or for use in targeting virus-like particles or lipid nanoparticles.

With the nanobody of the invention, AAV, Adenovirus or lentiviral vectors may be redirected to CD4+ cells, in particular CD4+ T cells. In Adeno-associated virus (AAV), the viral capsid, *inter alia*, defines viral tropism. Currently, in primates, thirteen AAV serotypes have been identified, differing in the tissues and cells that they infect, although the serotypes usually display a rather broad tissue specificity. E.g., AAV2, which is the best investigated serotype for gene transfer, is characterized by a broad tropism, infecting numerous cell types and tissues. Also, the deficit of all AAV serotypes to specifically and efficiently transduce peripheral blood mononuclear cells (PBMC) represents a considerable technical hurdle, in particular when direct *in vivo* gene transfer into human hematolymphoid cells is required. This drawback is now addressed and can be overcome with the nanobody of the invention, by means of which these cells, which express CD4, can be directly targeted.

In one embodiment, the nanobody of the invention, or the nucleic acid coding for the nanobody, is used for genetic labelling of AAV capsid proteins, preferably for genetic labeling of the AAV capsid proteins VP1 and/or VP2.

With the nanobody of the invention, e.g. AAV vector transduction of human CD4+ cells can be improved, including primary human T lymphocytes, by means of which, e.g. recombinases or nucleases can be introduced.

According to another aspect, the invention also concerns a multiparatopic, preferably a biparatopic, a multivalent, preferably a bivalent or trivalent, construct comprising at least one nanobody of the invention, or a combination thereof.

A "multiparatopic construct" as used herein and as generally understood refers to a polypeptide construct that binds to two or more different epitopes on one antigen, e.g. two (= biparatopic) or three (= triparatopic). Accordingly, the present invention also comprises constructs comprising the binding sequences of at least two of the nanobodies of the invention.

A "multivalent construct", on the other hand, is a construct that has at least two antigen binding sites/binding units. In some embodiments, a multivalent construct is a bivalent construct, trivalent construct or a tetravalent construct.

The present invention also concerns a method for determining and/or imaging and/or monitoring the presence and/or amount and/or activity or differentiation state of CD4+ cells in a biological sample suspected of comprising CD4+ cells, preferably CD4+ T cells, the method comprising the steps of:
a) combining said sample with at least one of the nanobodies of the present invention, or with at least one construct of the present invention, and optionally with at least one second compound conjugated with a detectable label, the second compound binding to another marker of the CD4+ cells, preferably CD4+ T cells,
b) measuring, via the detectable label, imaging signals from said nanobody that has bound to CD4+ cells, preferably CD4+ T cells, in said sample, and, if a second compound has additionally been used in step a), from said second compound that has bound to another marker; and
c) imaging, determining and/or monitoring the presence and/or amount and/or activity of CD4+ cells, preferably CD4+ T cells, in said sample via the imaging signals,
wherein the determination and/or monitoring and/or imaging is preferably for patient stratification and for monitoring individual immune responses during personalized immunotherapy.

A "biological sample" as used herein is meant to be a portion of a larger biological element. Preferably, the sample is a substance of biological origin. Examples of biological samples include, but are not limited thereto, portions of organs or tissues such as the kidney, the liver, heart, the lung, etc., the arteries, the veins, etc., the blood, e.g. whole blood, and its compounds such as the plasma, serum, the platelets, the subpopulations of blood cells etc., as well as saliva, sputum, or any sample retrieved from the mouth and pharynx region, also e.g. by using a mouth wash or rinse containing saliva or sputum samples. The biological sample, preferably, is from a mammal, preferably a human. Also, preferably, the biological sample can be a cell culture comprising cells derived from a mammal, preferably a human, such as primary cells or (established) cell lines, with a "primary cell culture" being the culture of cells directly isolated from a tissue of interest and retaining the morphological and functional characteristics of their tissue of origin. Whereas cell lines are cells that have been continually passaged over a long period of time and have acquired homogenous genotypic and phenotypic characteristics; they can be finite or continuous.

The nanobodies of the invention can not only be used on/in connection with CD4+ T cells, but also on other CD4 expressing cells, e.g. immune cells such as monocytes, macrophages, and dendritic cells. Accordingly, in one embodiment of the present invention, the CD4-expressing cell is selected from T-cells, monocytes, macrophages, and dendritic cells.

The present invention also concerns the use of at least one of the nanobodies of the present invention, or of at least one construct of the present invention, as a diagnostic agent, preferably wherein the diagnostic agent is used in a diagnosis which is selected from diagnostic classification of a disease-associated immune status of a patient, and susceptibility to immunotherapies of a patient.

The present invention also concerns a method for diagnosing and/or monitoring the immune status and/or susceptibility to immunotherapies of a patient, wherein at least one nanobody of the present invention, or at least one construct of the present invention,, is used to assess and/or monitor CD4+ T cells in a biological sample of the patient, wherein the nanobody is used alone or in combination with a second compound binding to a T-cell specific marker, wherein the T cell-specific marker is selected from at least one of CD2, CD3, CD7, CD8, CD27, CD28, CD127, HLA-DR, CD38, CD69, CCR4, CCR5, CCR6, CCR7, CTLA-4, LAG3, TIM-3, OX40, ICOS, CXCR4, PD-1, PD-L1, PD-L2, CD40L (synonym CD154), CD122, CD137, GITR, CD25, CD278, SIGLEC-7, SIGLEC-9, BTLA (synonym: CD272), TIGIT, VISTA, B7-H4 (synonym: VTCN1), CD276 (synonym: B7-H3), A2AR, CEACAM1, LAIR-3, HVEM, CD160, CD200, CD200R.

The present invention also concerns a pharmaceutical composition comprising at least one of the nanobodies of the invention and/or at least one construct of the invention, in association with a pharmaceutically acceptable carrier or excipient.

The composition of the invention comprises at least one nanobody of the invention, formulated for administration to/ for use with a patient in need of treatment with the composition/the nanobody of the invention.

The term "pharmaceutically" or "pharmaceutically acceptable" refers to molecular entities and compositions which do not produce secondary, allergic or other unfortunate reactions when they are administered to a mammal, in particular a human. Accordingly, a "pharmaceutically acceptable carrier" is understood to mean any excipient, additive, or vehicle that is typically used in the field of the treatment of diseases and which simplifies or enables the administration of the product according to the invention to a living being, preferably a human, and/or improves its stability and/or activity. The pharmaceutical composition can also incorporate binding agents, diluting agents or lubricants. The selection of a pharmaceutical carrier or other additives can be made on the basis of the intended administration route and standard pharmaceutical practice. As pharmaceutical acceptable carrier use can be made of/comprise one or more of solvents, extenders, or other liquid binding media such as dispersing or suspending agents, surfactant, isotonic agents, spreaders or emulsifiers, preservatives, encapsulating agents, solid binding media, lyoprotectants, bulking agents, stabilizer, depending upon what is best suited for the respective dose regime and is likewise compatible with the compound according to the invention. An overview of such additional ingredients can be found in, for example, Rowe (Ed.) et al.: Handbook of Pharmaceutical Excipients, 7th edition, 2012, Pharmaceutical Press.

The pharmaceutical composition can, e.g., comprise an aqueous buffer at a physiologically acceptable pH (e.g., pH 7 to 8.5), a polymer-based nanoparticle vehicle, a liposome, and the like. The pharmaceutical compositions can be delivered in any suitable dosage form, such as a liquid, gel, solid, cream, or paste dosage form.

Any administration method, known to one skilled in the art, may be used for administrating the nanobody or the pharmaceutical composition according to the invention to the patient. In particular, the nanobody/the pharmaceutical composition may be for example administered orally, by inhalation or via a parenteral route, in particular by injection, e.g., subcutaneous, intravascular, intramuscular or intraperitoneal. When the parenteral route is selected, the nanobody/pharmaceutical composition may be in the form of injectable solutions and suspensions, packaged in ampoules or flasks. The parenteral administration forms are conventionally obtained by mixing the nanobody according to the invention with buffers, stabilizers, preservatives, solubilizing agents, isotonic agents and suspending agents. According to known techniques, these mixtures may be sterilized or packaged as intravenous injections. One skilled in the art may for example use a buffer based on phosphate salts as buffers.

A "lyoprotectant" is a molecule which, when combined with a protein of interest, significantly prevents or reduces chemical and/or physical instability of the protein upon lyophilization and subsequent storage. Exemplary lyoprotectants include sugars such as sucrose, sorbitol, or trehalose; an amino acid such as monosodium glutamate or histidine; a methylamine such as betaine; a lyotropic salt such as magnesium sulfate; a polyol such as trihydric or higher sugar alcohols, e.g. glycerin, erythritol, glycerol, arabitol, xylitol.

A "stabilizer" refers to a molecule which, when combined with a protein of interest (e.g., the nanobdy/single domain antibody molecule) substantially prevents or reduces chemical and/or physical instability of the protein of interest in lyophilized, reconstituted, liquid or storage form. Exemplary stabilizers include sucrose, sorbitol, glycine, inositol, sodium chloride, methionine, arginine, and arginine hydrochloride.

The "diluent" of interest herein is one which is pharmaceutically acceptable (safe and non-toxic for administration to a human) and is useful for the preparation of a reconstituted formulation. Exemplary diluents include sterile water, bacteriostatic water for injection (BWFI), a pH buffered solution (e.g. phosphate-buffered saline), sterile saline solution, Ringer's solution or dextrose solution.

A "preservative" is a compound which can be added to the diluent to essentially reduce bacterial action in the reconstituted formulation, thus facilitating the production of a multi-use reconstituted formulation, for example. Examples of potential preservatives include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols such as phenol, butyl and benzyl alcohol, alkyl parabens such as methyl or propyl paraben, catechol, resorcinol, cyclohexanol, 3-pentanol, and m-cresol. The most preferred preservative herein is benzyl alcohol.

A "bulking agent" is a compound which adds mass to the lyophilized mixture and contributes to the physical structure of the lyophilized cake (e.g. facilitates the production of an essentially uniform lyophilized cake which maintains an open pore structure). Exemplary bulking agents include mannitol, glycine, polyethylene glycol and sorbitol.

The administered amount of nanobodies/the pharmaceutical composition naturally depends on the administration route/method, on the size and/or on the weight of the patient, on the detection technique used, and on the nature of the accessory moiety which may be associated therewith.

The pharmaceutical composition of the invention may include a surfactant, a bulking agent, a stabilizer, a preservative, and a buffer, e.g. a buffer for adjusting the pH of the composition. In some embodiments, the formulation is a liquid formulation, a lyophilized formulation, a reconstituted lyophilized formulation, an aerosol formulation, or a bulk storage formulation (e.g., frozen bulk storage formulation).

The patient preferably is a human.

The "biological sample" as used herein, is preferably selected from the group consisting of a tissue sample, body fluid sample, preferably a sputum sample and saliva sample, blood sample, preferably a whole blood sample, plasma sample or serum sample. The biological sample, as mentioned above preferably, is from a mammal, preferably a human. Also, preferably, the biological sample can be a cell culture comprising cells derived from a mammal, preferably a human, such as primary cells or (established) cell lines, with a "primary cell culture" being the culture of cells directly isolated from a tissue of interest and retaining the morphological and functional characteristics of their tissue of origin. Whereas cell lines are cells that have been continually passaged over a long period of time and have acquired homogenous genotypic and phenotypic characteristics; they can be finite or continuous.

By providing the amino acid sequence of the nanobody of the invention, one skilled in the art is capable of producing the nanobodies according to the invention and described herein, by conventional techniques for producing polypeptides. For example, they may be synthesized by using a well known synthesis method in a solid phase.

Alternatively, the nanobodies according to the invention may be synthesized with recombinant DNA techniques well known one skilled in the art (Maniatis et al. (1982) Molecular Cloning: a laboratory manual, Cold Spring Harbor Laboratories, NY, 51-54 and 412-430). For example, they may be obtained as DNA expression products after incorporating DNA sequences coding for the polypeptide of interest in expression vectors and introducing these vectors in suitable prokaryotic or eukaryotic hosts which will express the polypeptide of interest, from which they may then be isolated by using techniques well known to one skilled in the art. Accordingly, the invention also concerns a vector comprising a nucleic acid coding for the polypeptides and nanobodies as disclosed herein, as well as a transfected, infected or transformed cell with such a nucleic acid or vector.

Further advantages follow from the description of the embodiments and the attached drawings. All publications, patent applications, patents, and other references mentioned herein are incorporated by reference in their entirety.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description.

### BRIEF DESCRIPTION OF THE DRAWINGS

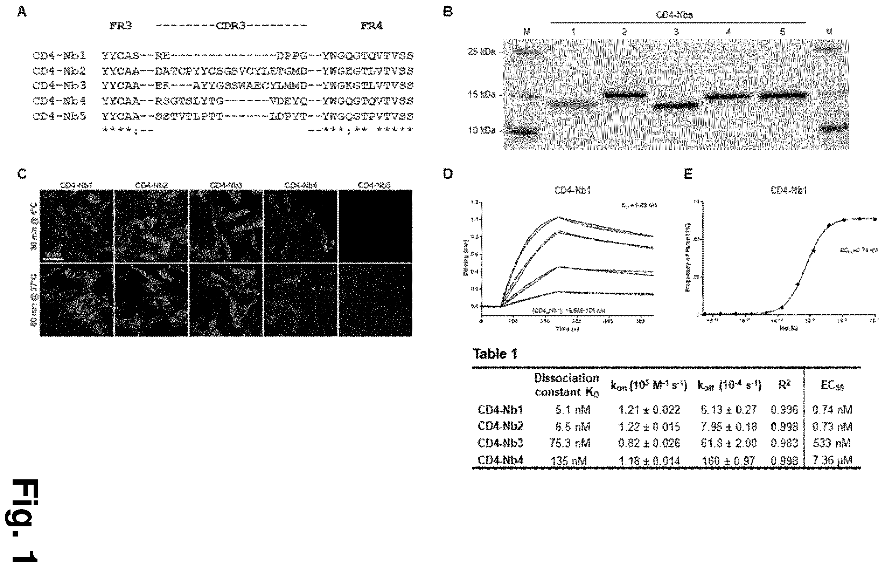

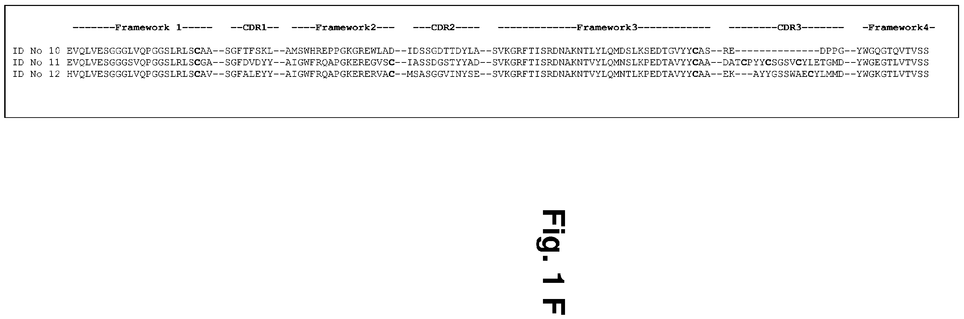

**Fig. 1** shows results of experiments for the identification and characterization of nanobodies against human CD4 of the invention; (**A**) Amino acid sequences of the complementarity determining region (CDR) 3 from unique CD4-Nbs selected after two rounds of biopanning are listed. (**B**) Recombinant expression and purification of CD4-Nbs using immobilized metal affinity chromatography (IMAC) and size exclusion chromatography (SEC). Coomassie staining of 2 µg of purified Nbs is shown. (**C**) Representative images of live CHO-hCD4 cells stained with CD4-Nbs for 30 min at 4°C (top row) or 60 min at 37°C (bottom row), scale bar: 50 µm. (**D**) For biolayer interferometry-based affinity measurements, biotinylated hCD4 was immobilized on streptavidin biosensors. Kinetic measurements were performed using four concentrations of purified Nbs ranging from 15.6 nM - 1000 nM. As an example, the sensogram of CD4-Nb1 at indicated concentrations is shown. (**E**) EC₅₀ determination by flow cytometry. Exemplarily shown for CD4-Nb1, the percentage of positively stained CHO-hCD4 (frequency of parent) was plotted against indicated concentrations of CD4-Nbs. **Table 1** Summary of affinities (K_{D}), association (Kₒₙ) and dissociation constants (K_{off}) determined by BLI (left side) and EC₅₀ values of flow cytometry (right side). (**F**) a sequence alignment of SEQ ID Nos. 16, 17 and 18.

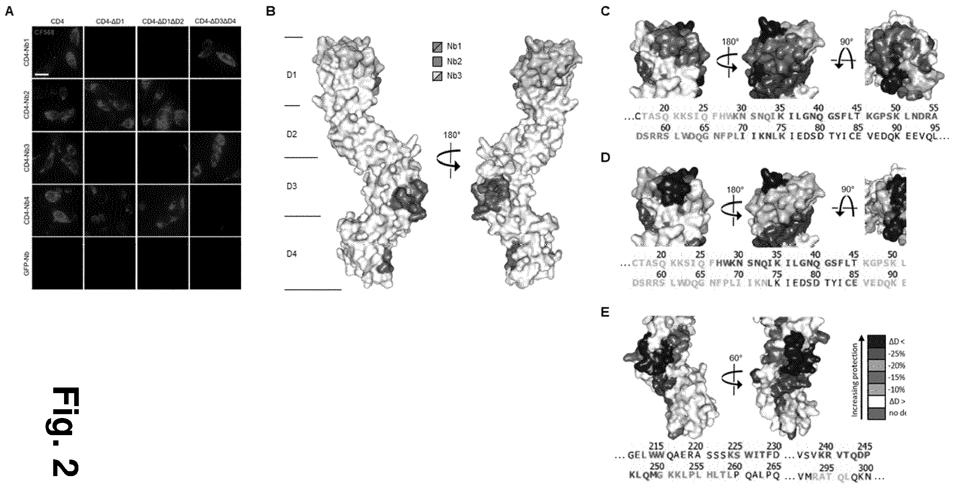

**Fig. 2** shows results of experiments of the localization of CD4-nanobodies binding epitopes; (**A**) Representative images of live CHO cells expressing full-length or domain-deletion mutants of hCD4 stained with fluorescently labeled CD4-Nbs (CF568). (**B**) Surface structure model of hCD4 (PDBe 1wiq) and the HDX-MS epitope mapping results of CD4-Nb1 - 3 are shown. Different colours highlight the amino acid residues protected by CD4-Nb1 (blue), CD4-Nb2 (red) or CD4-Nb 3 (yellow). Overlapping residues protected by both, Nb1 and Nb3 are coloured in green. A more detailed surface map (%ΔD) of these specific regions are shown in **C** (CD4-Nb1), **D** (CD4-Nb3) and **E** (CD4-Nb2) with the corresponding CD4 amino acid sequence.

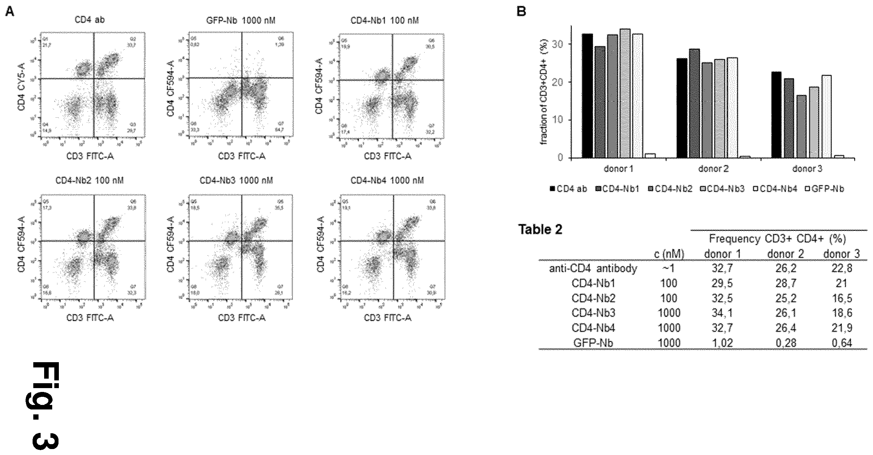

**Fig. 3** shows results of cytometry analyses of human PBMCs stained with fluorescently labeled CD4 nanobodies; (**A**) Schematic representation of the final gating step for CD3⁺CD4⁺ double-positive cells derived from donor one. (**B**) Percentage of double-positive cells of three donors, stained with CD4-Nb1 or CD4-Nb2 (100 nM), or CD4-Nb3 or CD4-Nb4 (1 µM), compared to anti-CD4 antibody and negative control Nb (GFP-Nb, 1 µM). **Table 2** Percentage of double-positive PBMCs from three donors stained with CD4-Nbs at indicated concentrations.

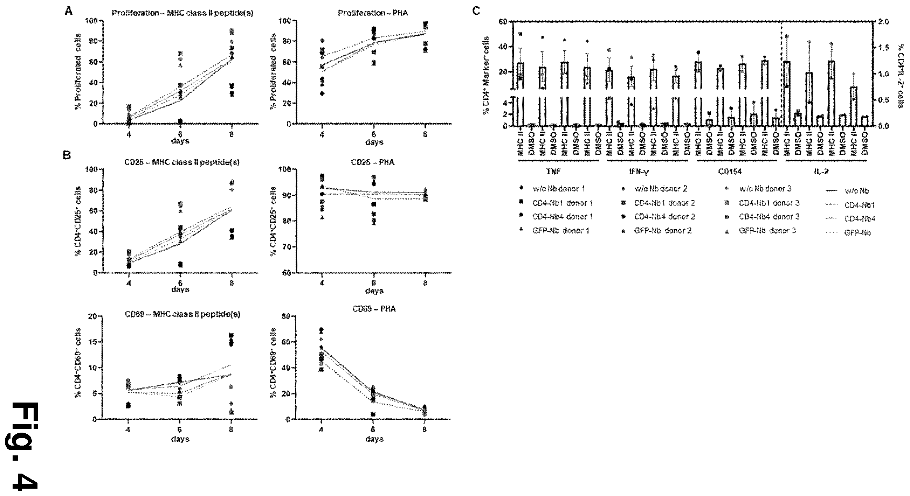

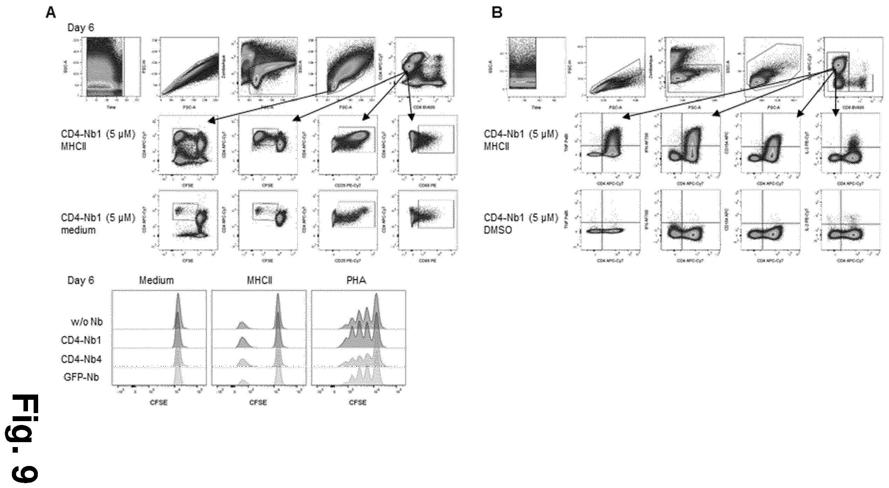

**Fig. 4** shows experiments for analyzing the impact of CD4 nanobodies on activation, proliferation and cytokine release of T cells; Cells were stained with CFSE, treated with 5 µM Nbs or w/o for 1 h (one replicate each), washed and then stimulated with 5 µg/ml MHC-class II peptides, 10 µg/ml PHA or not stimulated, and cultured for 12 days. (**A**) Cells were analyzed by flow cytometry for proliferation (CFSE-negative fraction) and activation (CD25 and CD69) on days 4, 6, 8. Proliferation of CD4⁺ cells after stimulation with MHC-class II peptide(s) (left) or PHA (right). (**B**) Activation markers on CD4⁺ cells, Top: CD25 expression after stimulation with MHCII peptide(s) (left) or PHA (right); Bottom: CD69 expression after stimulation with MHCII peptide(s) (left) or PHA (right). Mean percentages of all 3 donors are shown as plain or dotted lines. (**C**) Cytokine and activation marker expression of CD4⁺ cells - TNF, IFN-y, CD154 (left y-axis) or IL-2 (right y-axis). Cells were restimulated on day 12 with MHC-class II peptide(s) or DMSO (background) for 14h in the presence of Golgi Stop and Brefeldin A and analyzed by flow cytometry. Error bars display SEM. Gating strategy is shown in Fig. 9 below, all percentages are given within CD4⁺ T cells.

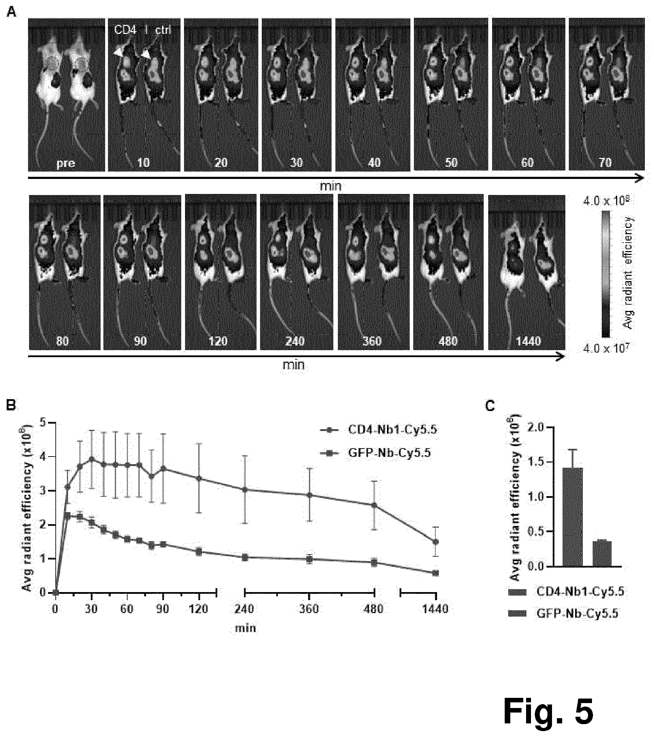

**Fig. 5** shows results of optical imaging (OI) experiments of CD4 nanobody CD4-Nb1-Cy5.5; 5 µg of CD4-Nb1-Cy5.5 or GFP-Nb-Cy5.5 were administered *i.v.* to s.c. human CD4⁺ HPB-ALL-bearing NSG mice and tumor bio distribution was monitored by repetitive OI measurements over the course of 24 h. (**A**) Representative images of each measurement time point of one mouse injected with CD4-Nb1-Cy5.5 (left, CD4) or GFP-Nb-Cy5.5 (right, ctrl). Red circles and white arrows indicate the tumor localization at the right upper flank. (**B**) Quantification of the fluorescence signal from the tumors (n = 4 per group, arithmetic mean of the average radiant efficiency ± SEM). (**C**) After the last imaging time point tumors were explanted for *ex vivo* OI, confirming increased accumulation of CD4-Nb1-Cy5.5 compared to the GFP-Nb-Cy5.5 (n = 2 per group, arithmetic mean ± SEM);

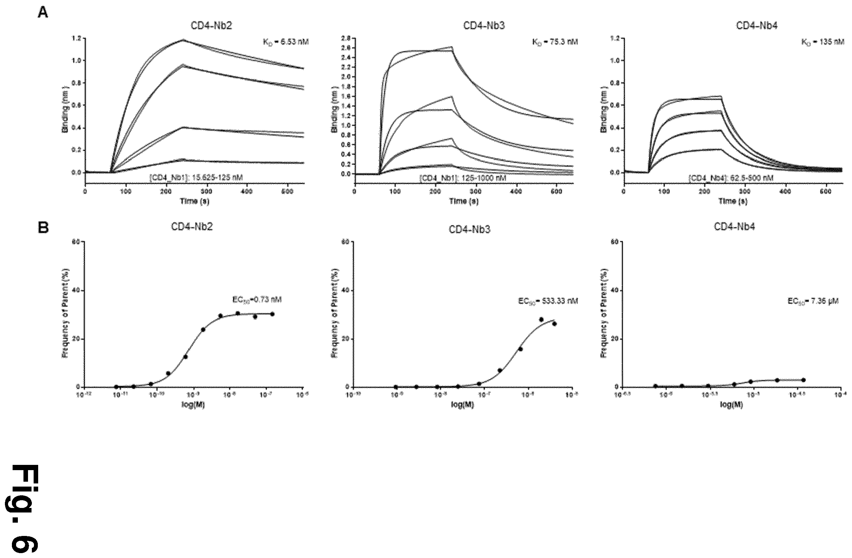

**Fig. 6** shows diagrams for the affinities of examples of the CD4-nanobodies of the invention; (**A**) Sensograms of biolayer interferometry-based affinity measurements of CD4-Nb2, CD4-Nb3 and CD4-Nb4 are shown. Biotinylated hCD4 was immobilized on streptavidin biosensors and kinetic measurements were performed by using four concentrations of purified Nbs ranging from 15.6 nM - 1 µM. (**B**) EC₅₀ determination of CD4-Nbs for cellularly expressed hCD4 by flow cytometry. The percentage of positively stained CHO-hCD4 (frequency of parent) was plotted against indicated concentrations of CD4-Nbs. EC₅₀ values were calculated from a four-parametric sigmoidal model;

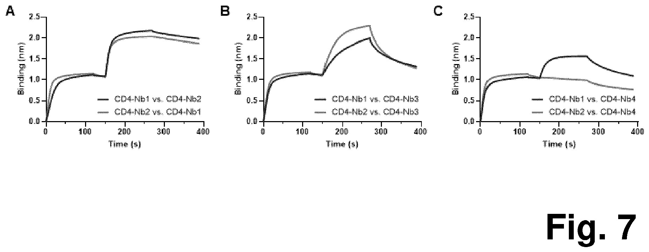

**Fig. 7** shows diagrams for the epitope binning analysis of examples of the CD4 nanobodies of the invention by biolayer interferometry (BLI); (**A**) Representative BLI sensograms of single measurements of combinatorial Nb binding to the recombinant extracellular portion of human CD4 of CD4-Nb1 (darker) and CD4-Nb2 (lighter) with (**A**) one another, (**B**) CD4-Nb3, or (C) CD4-Nb4; (D) a sequence alignment of SEQ ID Nos. 16, 17 and 18.

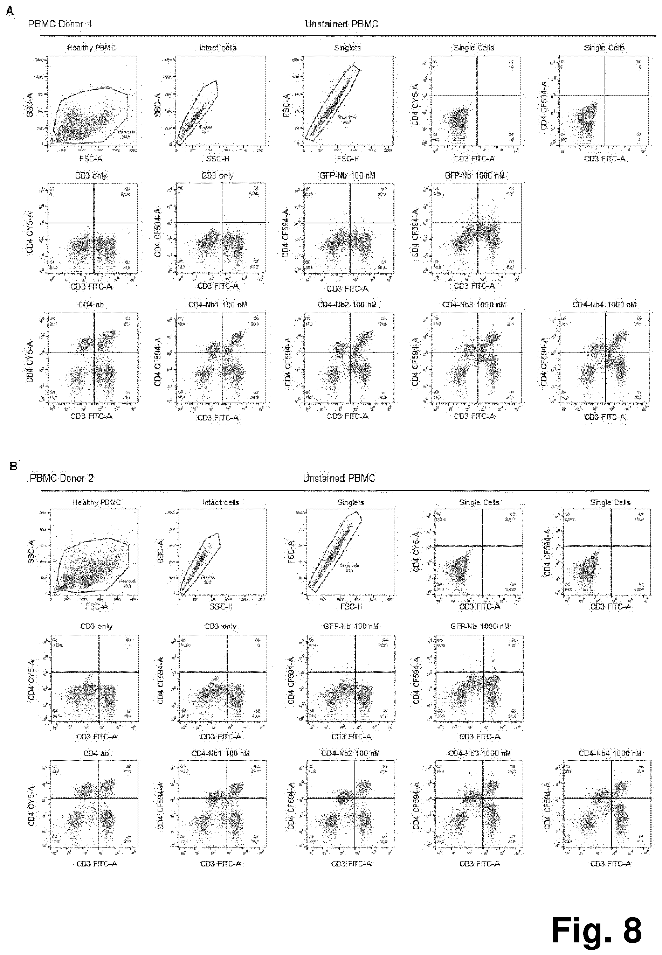

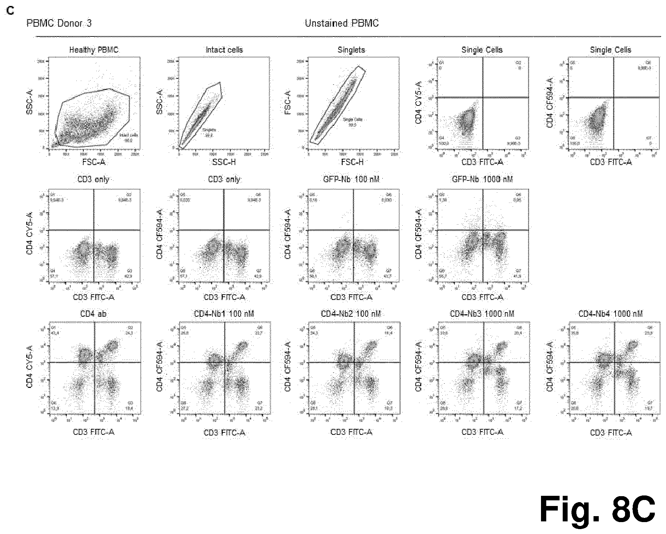

**Fig. 8** shows diagrams for the results of binding studies of CD4-nanobodies of the invention to CD4⁺ cells present in human PBMCs. Top row shows gating strategy for flow cytometry analysis of CD4⁺CD3⁺ double-positive human PBMCs. Middle and bottom row shows final gating step and quantification of these cells for donor 1 (**A**), donor 2 (**B**), and donor 3 (**C**) stained with an anti-CD4 antibody (CD4 ab), anti-GFP control Nb (GFP-Nb), or CD4-Nb1 - CD4-Nb4 at indicated concentrations;

**Fig. 9** shows results of the determination of the effect of CD4-nanobodies on CD4⁺ T cells; **A**) Gating strategy for analysis of proliferation and activation of CD4⁺ cells after stimulation. Top row from left to right: Time gate, single cells, live cells, lymphocytes, CD4⁺ cells. Middle and bottom rows: gates were placed on proliferating CFSE-negative CD4⁺ cells (left), CD25⁺CD4⁺ cells (middle) and CD69⁺CD4⁺ cells (right). Histogram overlay shows the number of divisions as CFSE labeling within CD4⁺ cells. Shown is one representative example (donor 2) on day 6. (**B**) Gating strategy (donor 3) for analysis of activation marker and cytokine expression of CD4⁺ cells in intracellular staining after 12 days of culture and 14 h re-stimulation. Top row from left to right: Time gate, single cells, live cells, lymphocytes, CD4⁺ cells, CD4/CD8 staining (gating on CD8^{neg} cells). Middle and bottom rows show the expression of TNF, IFN-γ, CD154 and IL-2 after re-stimulation with MHC-class II peptides or control DMSO/water.

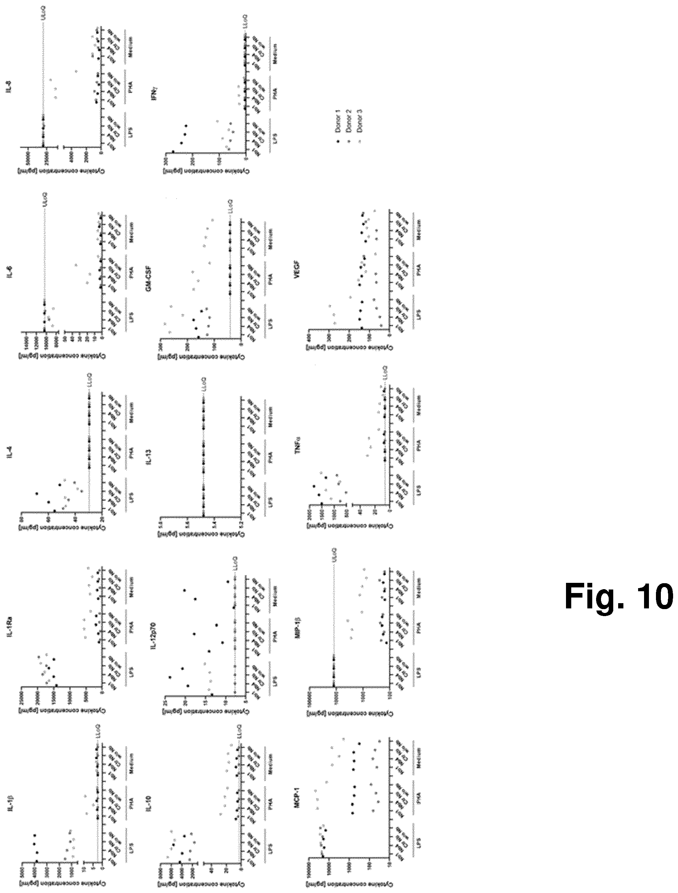

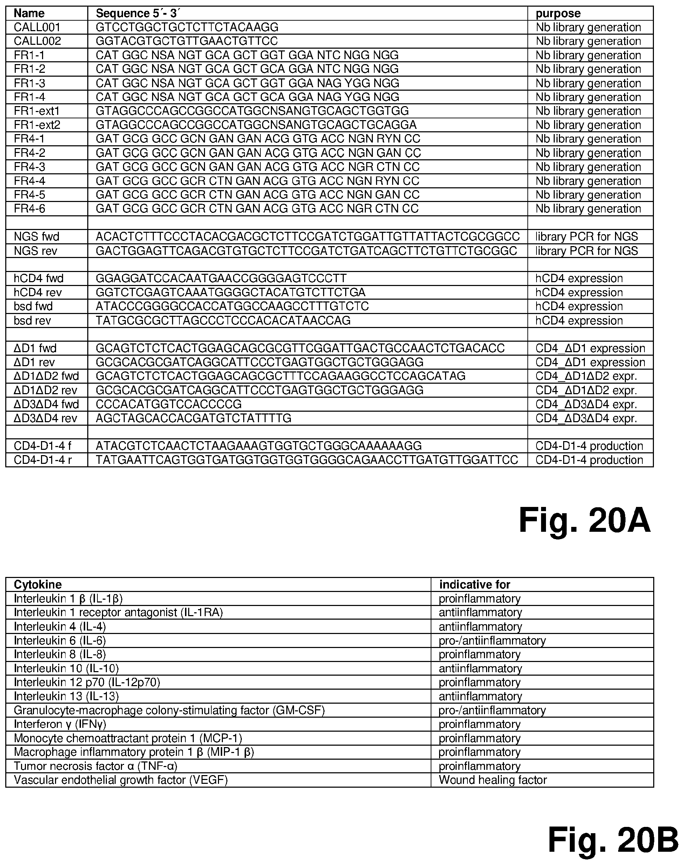

**Fig. 10** shows results of the determination of cytokines secreted from whole blood samples of donors after treatment with CD4-Nbs. Blood samples of three donors were incubated with 5 µM CD4-Nb1, CD4-Nb4, GFP-Nb (control) or w/o Nb and stimulated with lipopolysaccharide (LPS), phytohaemagglutinin (PHA) or medium only as control. Secreted cytokines (listed in Fig. 20B) were measured and quantified using an in housed developed microsphere-based (Luminex) multiplex sandwich immunoassay. Results of one biological experiment are shown as colored dots indicating measured cytokine levels of one individual.

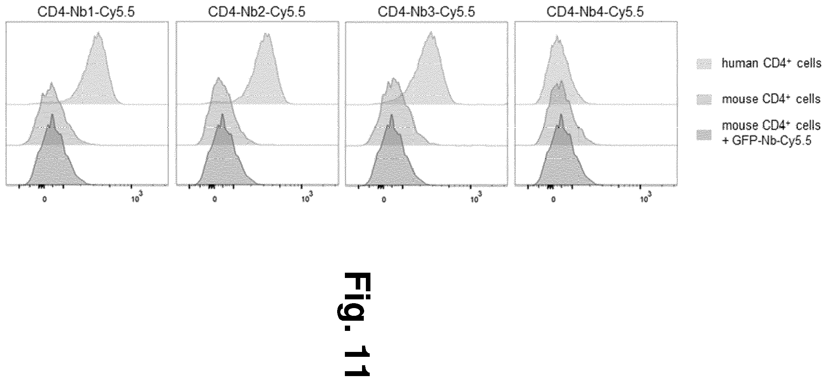

**Fig. 11** shows results of cross-species reactivity testing of Cy5.5-labeled CD4-nanobodies of the invention; Flow cytometry of human and mouse CD4⁺ cells stained with CD4-Nbs-Cy5.5 or GFP-Nb-Cy5.5. Binding to human CD4 was confirmed for CD4-Nb1-3. The low-affinity CD4-Nb4 did not show staining at this concentration (0.75 µg/ml, -49 nM). None of the nanobodies stained murine CD4;

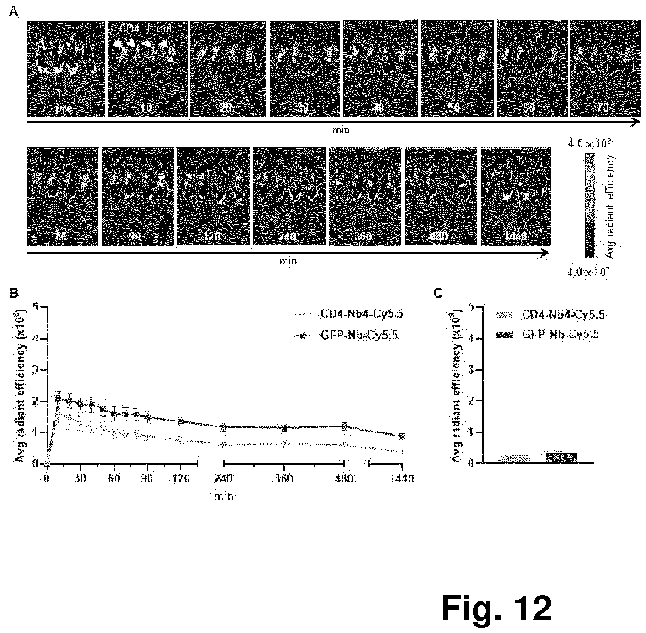

**Fig. 12** shows results of *in vivo* optical imaging (OI) of low-affinity binding CD4-Nb4-Cy5.5; 5 µg of CD4-Nb4-Cy5.5-or GFP-Nb-Cy5.5 were administered i.v. to s.c. human CD4+ HPB-ALL-bearing NSG mice and tumor bio distribution was monitored by repetitive OI measurements over the course of 24 h. (**A**) Representative images of each measurement time point of 4 mice injected either with CD4-Nb4-Cy5.5 (left, CD4) or GFP-Nb-Cy5.5 (right, ctrl). White arrows indicate the tumor localization at the right upper flank. (**B**) Quantification of the fluorescence signal from the tumors (n = 4 per group, arithmetic mean of the average radiant efficiency ± SEM). (**C**) After the last imaging time point, tumors were explanted for ex vivo OI, demonstrating similar accumulation of CD4-Nb4-Cy5.5 and GFP-Nb-Cy5.5 (n = 2 per group, arithmetic mean ± SEM);

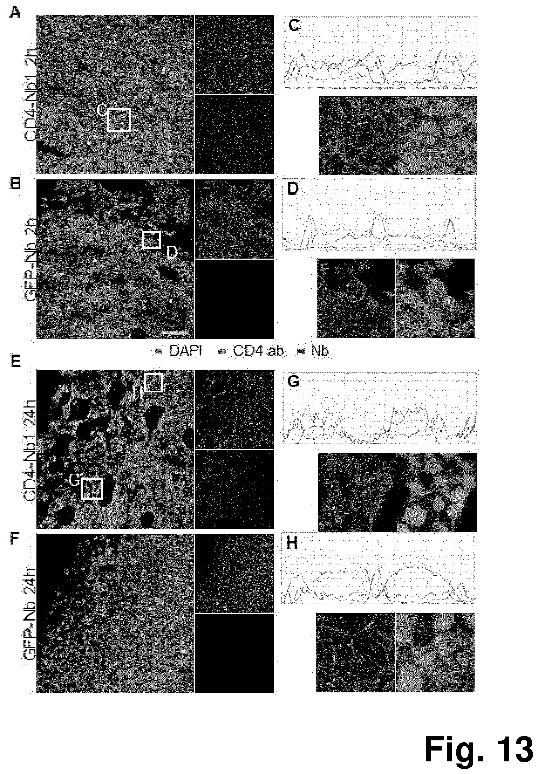

**Fig. 13** shows results of immunofluorescence staining experiments of ex *vivo* HPB-ALL tumors; Cryosections from ex *vivo* HPB-ALL tumors were imaged for bound Nb (blue) from the systemic injection in xenografted mice and co-stained with CD4-specific antibody (red) and nuclear DAPI staining (green). Image overlay of CD4-Nb1 (**A**) or control GFP-Nb (**B**) with CD4 antibody fluorescence after 2 hours of Nb injection. (**C**, **D**) Line-scan quantification of indicated image sections from A and B. (**E**, **F**) same as A and B after 24 h of systemic Nb injection. (**G**, **H**) Line-scan analysis of indicated image sections from E of strongly stained area (G) or weakly stained area (H).

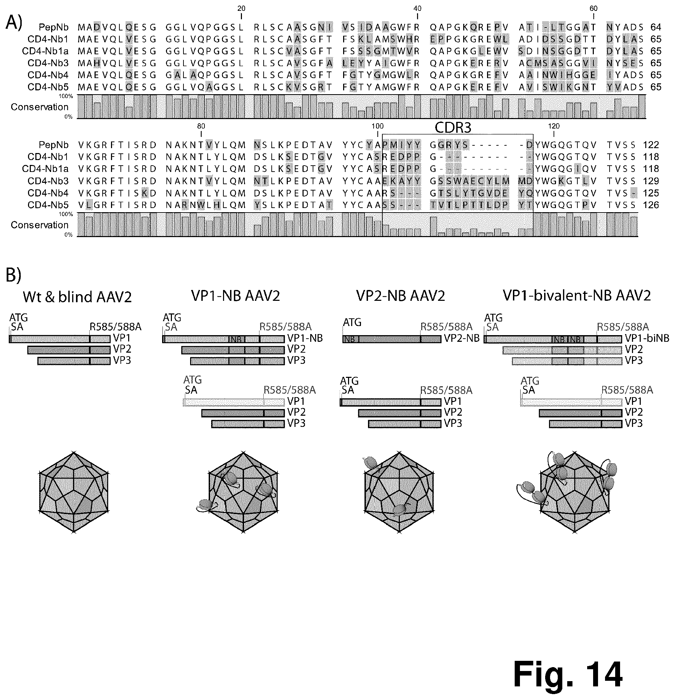

**Fig. 14** shows data concerning nanobody sequences and AAV2-nanobody design as exemplary used in the present invention: **A**) Amino acid alignment of the nanobodies used in this study. Nanobody annotation in accordance to the studies above, which describe a thorough characterization and application for *in vivo* imaging. Nb (nanobody) 1a shares the identical complementary determining region 3 (CDR3) as Nb1 (amino acids 101-117 in the alignment). **B**) Plasmids constructs used for AAV2 particle production and schematic representation of resulting capsids. Equimolar amounts of plasmids were co-transfected into HEK293T cells. For clarity, AAV helper plasmid and vector genome encoding a GFP reporter included in all co-transfections is not visualized here. Essential changes to optimized viral capsid proteins VP1-3 are indicated - lighter: mutated / black: wt. ATG - start codon; SA - splice acceptor site; NB - Nanobody; R585A/R588A - Arg to Ala amino acid substitution at residues 585 and 588 (blind), respectively.

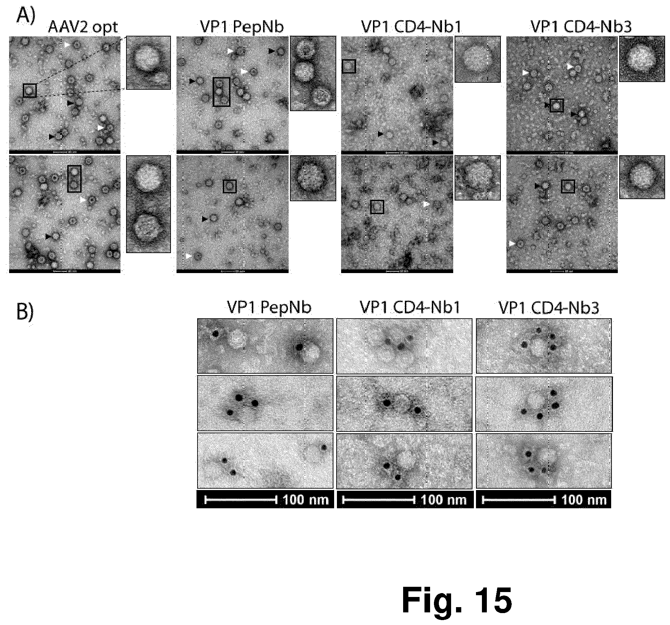

**Fig. 15** shows results of the electron microscopic analysis of nanobody-containing AAVs capsids: **A)** Purified AAV2 particles were subjected to negative staining electron microscopy. Representative images show vector genome-containing (black arrow) and empty (white arrow) capsids. Individual capsids are magnified. Scale bar 50 nm. **B)** Immuno-gold staining of purified AAV2 particles indicates accessible nanobodies at the capsid surface. Images were cropped to show representative capsids. Scale bar 100 nm.

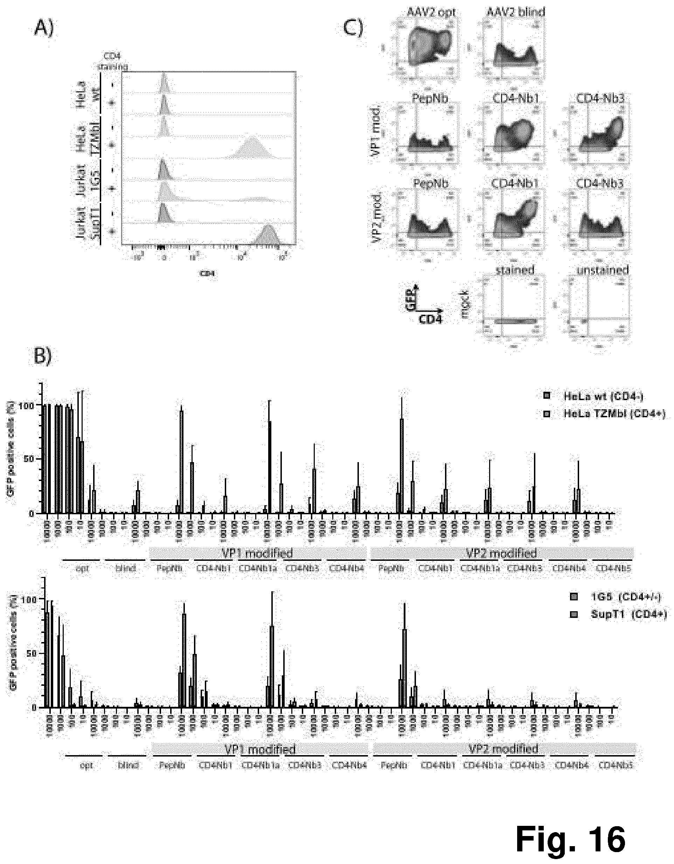

**Fig. 16** shows results on transduction experiments of CD4 expressing cells with AAV2 CD4-nanobody containing particles: **A**) CD4 expression on cell lines used in this study. Cells were stained with anti-CD4-Cy7 antibody and compared to unstained mock controls. The CD4 signal in mock cells was manually adjusted to 100 au and compared to stained cells by flow cytometry. Histograms are normalized by modal mode to visualize comparable peak heights. **B**) Two HeLa (wt and TZMbl) and two T lymphoid (1G5 and SupT1) cell lines were transduced with indicated viral genomes copies per cell (gc/cell). Three days post infection % GFP positive cells was determined by flow cytometry; n=2-4. Error bars indicate SD. **C**) 1G5 cells were transduced with indicated AAV2 particles at a concentration of 10.000 gc/cell. Three days post transduction cells were stained for CD4 expression and analyzed for GFP expression by flow cytometry. Representative data are shown;

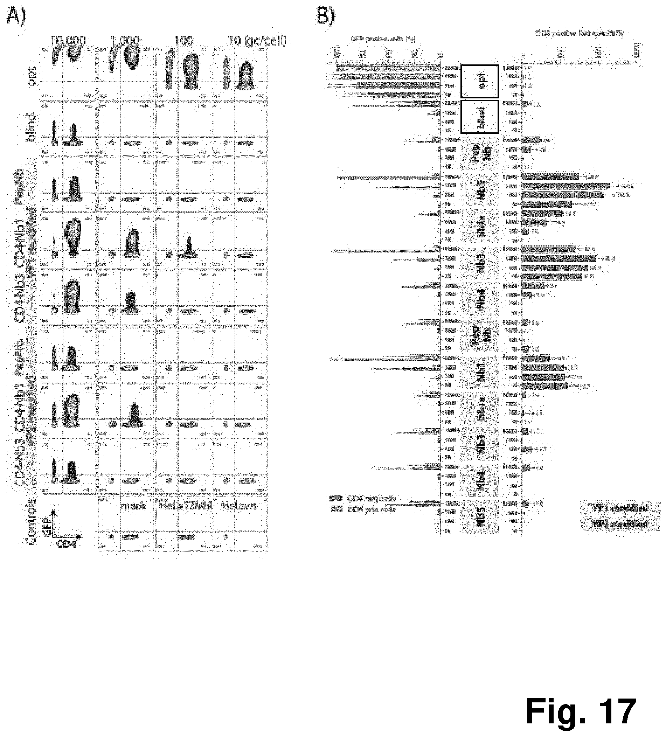

**Fig. 17** shows results of nanobody specificity in mixed culture experiments: **A**) Representative analysis of a mixed culture experiment. HeLa wt (CD4 negative) were mixed with HeLa TZMbl (CD4 positive) in a ratio of 1:1 prior to AAV2 transduction and subsequently transduced in different virus dilutions. Three days post transduction, cells were harvested, stained for CD4 and analyzed for GFP expression by flow cytometry. **B** Cumulative data from independent HeLa mixed culture experiments. Indicated are relative frequencies of GFP positive cells for CD4 positive and negative cells, respectively (left panel in B). AAV2 CD4-specific transduction is calculated as ratio from the individual cell populations (fold CD4 positive over CD4 negative). Fold changes are indicated on the right; n = 2-6, presented are means with SD.

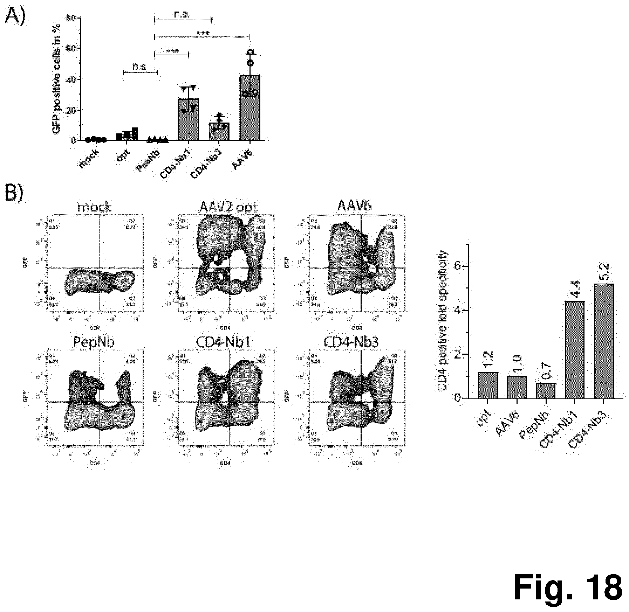

**Fig. 18** shows results for experiments analyzing the AAVS VP1 CD4 nanobody specificity in primary CD4+ T and PBMC; **A**) Primary human CD4+ T cells from two healthy donors were CD3/CD28 activated and transduced with indicated virus stocks (20.000 gc/cell). Three days post transduction, cells were stained for CD4 and analyzed for GFP expression by flow cytometry. Error bars indicate SD; n=4. **B**) Primary human PBMC were CD3/CD28 stimulated and subsequently infected with indicated virus stocks (10.000 gc/cell). Three days post infection, cells were stained for CD4 and GFP expression was assessed via flow cytometry (left panel). Fold specificity for CD4 positive cells (right panel) was determined from relative GFP positive populations in CD4 negative and positive cells, respectively. Data from one representative experiment.

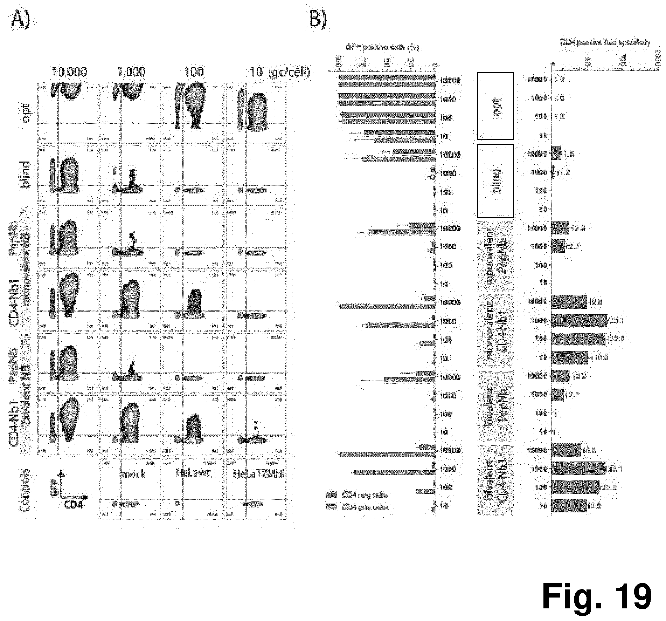

**Fig. 19** shows results of AAV2-VP1 bivalent-CD4 nanobody specificity in mixed culture experiments; **A**) Representative analysis of a mixed culture experiment comparing VP1-CD4-monovalent with bivalent nanobody constructs. HeLa wt (CD4 negative) were mixed with HeLa TZMbl (CD4 positive) in a ratio of 1:1 prior to AAV2 transduction and subsequently transduced with different virus dilutions. Three days post transduction cells were harvested, stained for CD4 and analyzed for GFP expression by flow cytometry. **B**) Summary of three independent HeLa mixed culture experiments. Indicated are relative frequencies of GFP positive cells for CD4 positive and negative cells, respectively (left panel). AAV2 CD4-specific transduction is calculated as ratio from the individual cell populations (fold CD4 positive over CD4 negative). Fold changes are indicated on the right; n=3, presented are means with SD.

**Fig. 20** shows two tables: **A)** primers used for and **B)** cytokines analyzed in the experiments presented herein under the title "use of CD4-nanobodies in *in vivo* imaging of human CD4".

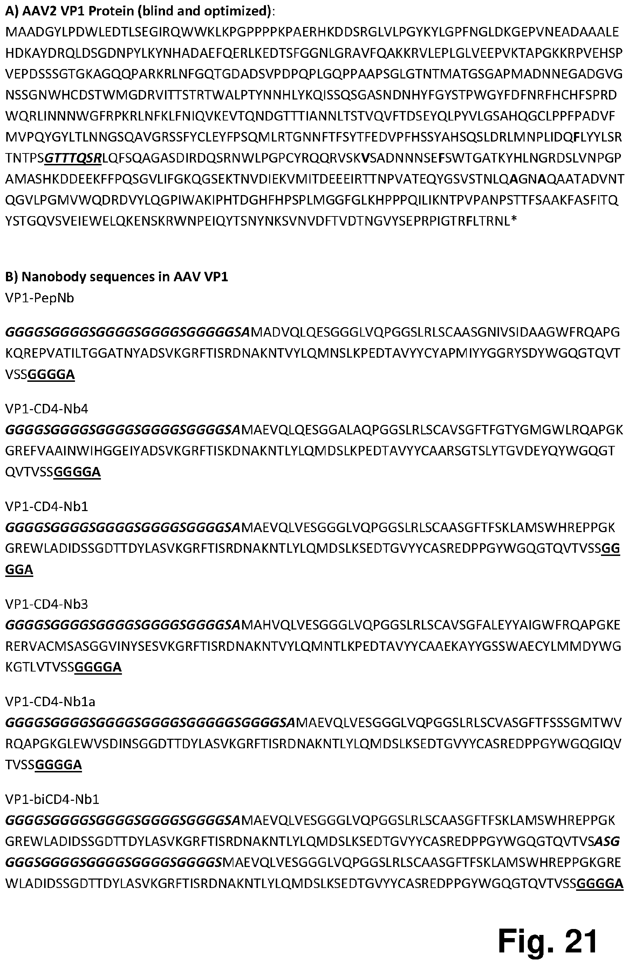

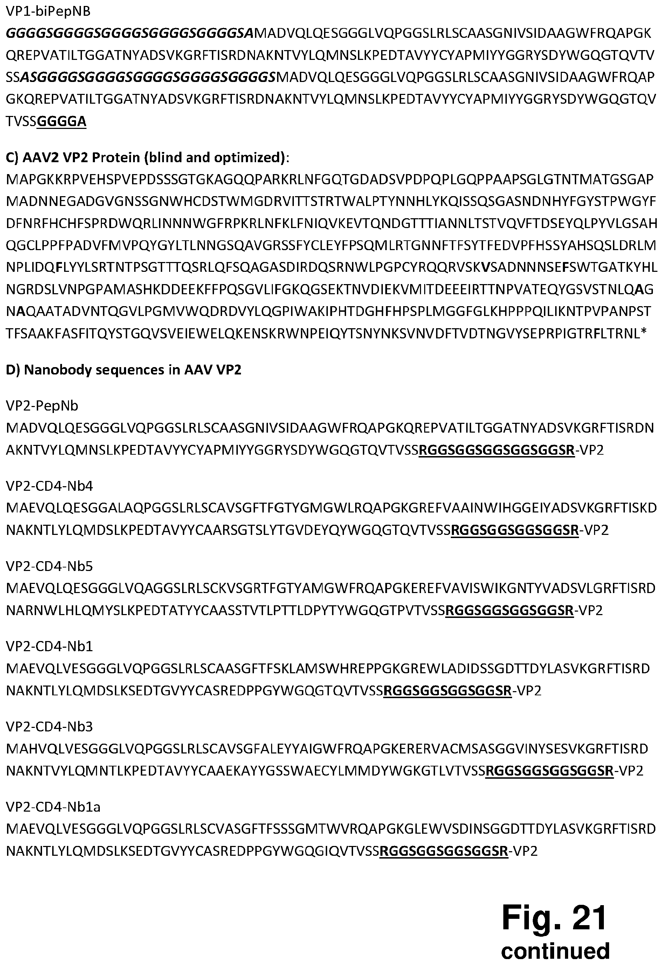

**Fig. 21** shows the amino acid sequences of exemplarily engineered AAV2 VP1 & VP2 proteins with CD4-specific nanobodies of the invention, with the following legend: A) AAV2 VP1 original sequence, Modified amino acids - in **bold,** Sequence replaced by nanobody insertion - in ***bold, underlined and italic;*** B) Nanobody sequences in AAV VP, N-terminal/central linker sequence - in ***bold & italic***, C-terminal linker sequence - in **bold and underlined**; C) AAV2 VP2 original sequence, Modified amino acids - in **bold;** D) Nanobody sequences in AAV VP2, C-terminal linker sequence - in **bold and underlined;**

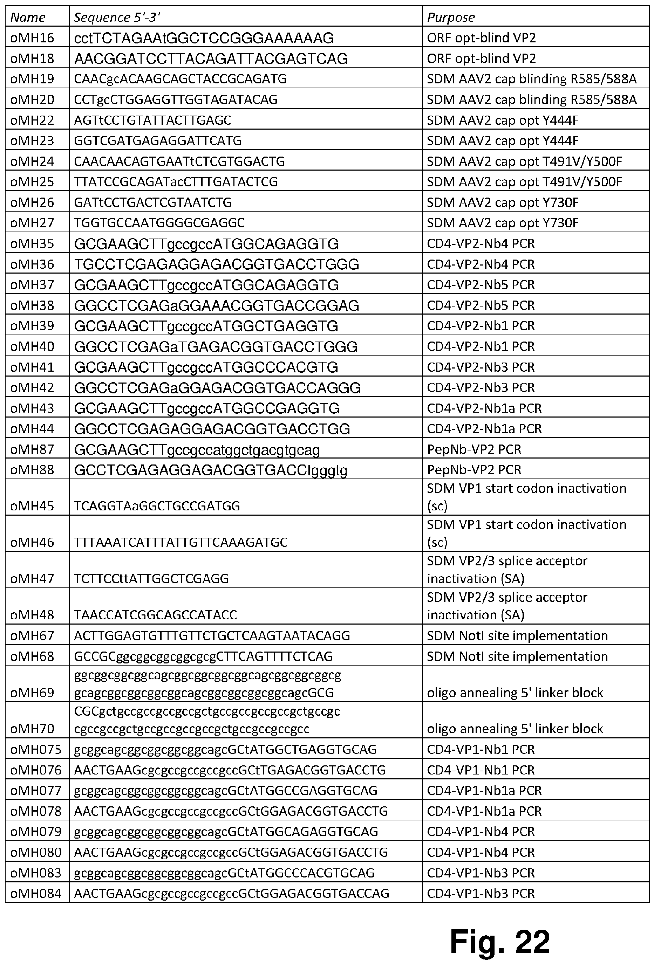

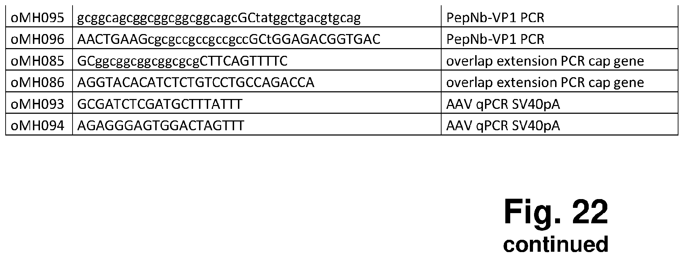

**Fig. 22** shows a table listing the oligonucleotides used in the experiments presented herein under the title "Targeting of CD4+ T cells by nanobody-modified AAV2 gene therapy vectors".

### DETAILED DESCRIPTION OF PREFERRED EMBODIMENTS

Within the present invention, a set of human CD4-specific nanobodies (in the following also: "Nbs") was generated, their binding properties on recombinant and cellular CD4 were examined, and their epitopes with sub-domain resolution were determined. A series of immunological analyses were performed to evaluate their effects on T-cell proliferation and activation and cytokine production in isolated human PBMCs and whole blood. Further, Nbs were converted into CD4 immunoprobes for optical imaging by site-directed fluorescent labeling and investigated their ability to visualize human CD4-positive tissue in a murine xenograft model by *in vivo* optical imaging.

### 1. Use of CD4-nanobodies in in vivo imaging of human CD4

### Materials and Methods

### Nanobody library generation

Alpaca immunization and Nb library construction were carried out as described previously. Animal immunization has been approved by the government of Upper Bavaria (Permit number: 55.2-1-54-2532.0-80-14). In brief, an alpaca (*Vicugna pacos*) was immunized with the purified extracellular domains of human CD4 (aa26-390) recombinantly produced in HEK293 cells (antibodies-online GmbH, Aachen, Germany). After initial priming with 1 mg the animal received six boost injections with 0.5 mg hCD4 each every second week. 87 days after initial immunization, -100 ml of blood were collected and lymphocytes were isolated by Ficoll gradient centrifugation using Lymphocyte Separation Medium (PAA Laboratories GmbH). Total RNA was extracted using TRIzol (Life Technologies) and mRNA was transcribed into cDNA using the First-Strand cDNA Synthesis Kit (GE Healthcare). The Nb repertoire was isolated in 3 subsequent PCR reactions using the following primer combinations (1) CALL001 and CALL002, (2) forward primer set FR1-1, FR1-2, FR1-3, FR1-4 and reverse primer CALL002, and (3) forward primer FR1-ext1 and FR1-ext2 and reverse primer set FR4-1, FR4-2, FR4-3, FR4-4, FR4-5 and FR4-6 introducing Sfil and Notl restriction sites. The Nb library was subcloned into the Sfil/ Notl sites of the pHEN4 phagemid vector.

### Nb Screening

For the selection of CD4-specific Nbs two consecutive phage enrichment rounds were performed, both with immobilized recombinant antigen and CHO-hCD4 cells. E.coli TG1 cells comprising the CD4-Nb-library in pHEN4 were infected with the M13K07 helper phage to generate Nbs presenting phages. For each round 1 × 10¹¹ phages of the CD4-Nb-library were applied on immunotubes coated with hCD4 (10 µg/ml). In each selection round extensive blocking of antigen and phages was performed with 5% milk or BSA in PBS-T and with increasing panning rounds PBS-T washing stringency was increased. Bound phages were eluted in 100 mM tri-ethylamine, TEA (pH 10.0), followed by immediate neutralization with 1 M Tris/HCI pH7.4. For cell-based panning, 2 × 10⁶ CHO-hCD4 or HEK293-hCD4 were non-enzymatically detached using dissociation buffer (Gibco) and suspended in 5% FCS in PBS. Antigen expressing cells were incubated with 1 × 10¹¹ phages under constant mixing at 4°C for 3 h. Cells were washed 3 × with 5% FCS in PBS. Phages were eluted with 75 mM citric acid buffer at pH2.3 for 5 min. To deplete non-CD4-specific phages, eluted phages were incubated 3 × with 1 × 10⁷ wt cells. Exponentially growing E.coli TG1 cells were infected with eluted phages from both panning strategies and spread on selection plates for following panning rounds. Antigen-specific enrichment for each round was monitored by counting colony forming unit (CFUs).

### Whole-cell phage ELISA

Polystyrene Costar 96-well cell culture plates (Corning) were coated with poly-L-lysine (Sigma Aldrich) and washed once with H₂O. CHO-wt and CHO-hCD4 were plated at 2 × 10⁴ cells per well in 100 µl and grown to confluency overnight. Next day, 70 µl of phage supernatant were added to culture medium of each cell type and incubated at 4°C for 3 h. Cells were washed 5 × with 5% FCS in PBS. M13-HRP-labeled antibody (Progen) was added at a conc. 0,5 ng/ml for 1 h, washed 3 × with 5% FCS in PBS. Onestep ultra TMB 32048 ELISA substrate (Thermo Fisher Scientific) was added and incubated until color change was visible and reaction was stopped by addition of 100 µl of 1M H₂SO₄. Detection occurred at 450 nm at a Pherastar plate reader and phage ELISA-positive clones were defined by a 2-fold signal above wt control cells.

### Expression constructs

The cDNA of human CD4 (UniProtKB - P01730) was amplified from hCD4-mOrange plasmid DNA (hCD4-mOrange was a gift from Sergi Padilla Parra; addgene plasmid #110192; http://n2t.net/addgene:110192; RRID:Addgene_110192) by PCR using forward primer hCD4 fwd and reverse primer hCD4 rev and introduced into BamHI and Xhol sites of a pcDNA3.1 vector variant (pcDNA3.1(+)IRES GFP, a gift from Kathleen_L Collins; addgene plasmid #51406; http://n2t.net/addgene:51406; RRID:Addgene_51406). The neomycin resistance gene (NeoR) was replaced with the cDNA for Blasticidin S-deaminase (bsd), amplified with forward primer bsd fwd and reverse primer bsd rev, by integration into the Xmal and BssHII sites of the vector. CD4 domain deletion mutants were generated using the Q5 Site-Directed Mutagenesis Kit (NEB) according to the manufacturer's protocol. For mutants lacking domain D1 of hCD4 an N-terminal BC2 tag was introduced. For the generation of plasmid pcDNA3.1_CD4_ΔD1_IRES-eGFP forward primer ΔD1 fwd and reverse primer ΔD1 rev were used; for pcDNA3.1_CD4_ΔD1ΔD2_IRES-eGFP forward primer ΔD1ΔD2 fwd and reverse primer ΔD1ΔD2 rev; for pcDNA3.1_CD4_ΔD3ΔD4_IRES-EGFP forward primer ΔD3ΔD4 fwd and reverse primer ΔD3ΔD4 rev. For bacterial expression of Nbs, sequences were cloned into the pHEN6 vector, thereby adding a C-terminal sortase tag LPETG followed by 6xHis-tag for IMAC purification as described previously. For protein production of the extracellular domains D1-4 of hCD4 in Expi293 cells, corresponding cDNA was amplified from plasmid DNA containing full-length human CD4 cDNA (addgene plasmid #110192) using forward primer CD4-D1-4 f and reverse primer CD4-D1-4 r. A 6xHis tag was introduced by the reverse primer. Esp3I and EcoRI restriction sites were used to introduce the cDNA into a pcDNA3.4 expression vector with the signal peptide MGWTLVFLFLLSVTAGVHS from the antibody JF5.

### Cell Culture, Transfection, Stable Cell Line Generation

HEK293T and CHO-K1 cells were obtained from ATCC (CCL-61, LGC Standards GmbH, Wesel, Germany). As this study does not include cell line-specific analysis, cells were used without additional authentication. Cells were cultivated according to standard protocols. Briefly, growth media containing DMEM (HEK293) or DMEM/F12 (CHO) (both high glucose, pyruvate, Thermo Fisher Scientific (TFS)) supplemented with 10% (v/v) fetal bovine serum, L-glutamine and penicillin-streptomycin (all from TFS) were used for cultivation. Cells were passaged using 0.05% trypsin-EDTA (TSF) and were cultivated at 37°C and 5% CO₂ atmosphere in a humidified chamber. Plasmid DNA was transfected using Lipofectamine 2000 (TSF) according to the manufacturer's protocol. For the generation of the stable HEK293-hCD4 and CHO-hCD4 cell line, 24 h post transfection, cells were subjected to a two-week selection period using 5 µg/ml Blasticidin S (Sigma Aldrich) followed by single cell separation. Individual clones were analyzed by live-cell fluorescence microscopy regarding their level and uniformity of GFP and CD4 expression.

### Protein expression and purification

CD4-specific Nbs were expressed and purified as previously published. Extracellular fragment of human CD4 comprising domains D1-4 of human CD4 and a C-terminal His6-tag was expressed in Expi293 cells according to the manufacturer's protocol (Thermo Fisher Scientific). Cell supernatant was harvested by centrifugation 4 days after transfection, sterile filtered and purified according to previously described protocols. For quality control, all purified proteins were analyzed via SDS-PAGE according to standard procedures. Therefore, protein samples were denaturized (5 min, 95°C) in 2x SDS-sample buffer containing 60 mM Tris/HCI, pH6.8; 2% (w/v) SDS; 5% (v/v) 2-mercaptoethanol, 10% (v/v) glycerol, 0.02% bromphenole blue. All proteins were visualized by InstantBlue Coomassie (Expedeon) staining. For immunoblotting proteins were transferred to nitrocellulose membrane (Bio-Rad Laboratories) and detection was performed using anti-His primary antibody (Penta-His Antibody, #34660, Qiagen) followed by donkey-anti-mouse secondary antibody labeled with AlexaFluor647 (Invitrogen) using a Typhoon Trio scanner (GE-Healthcare, Freiburg, Germany; excitation 633 nm, emission filter settings 670 nm BP 30).

### Live-cell immunofluorescence

CHO-hCD4, and CHO wt cells transiently expressing CD4 domain-deletion mutants were plated at ~10,000 cells per well of a µclear 96-well plate (Greiner Bio One, cat. #655090) and cultivated at standard conditions. Next day, medium was replaced by live-cell visualization medium DMEMgfp-2 (Evrogen, cat. #MC102) supplemented with 10% FCS, 2 mM L-glutamine, 2 µg/ml Hoechst33258 (Sigma Aldrich) for nuclear staining, and fluorescently labeled or unlabeled CD4-Nbs at concentrations between 1 nM and 100 nM. Unlabeled CD4-Nbs were visualized by addition of 2.5 µg/ml anti-VHH secondary Cy5 AffiniPure Goat Anti-Alpaca IgG (Jackson Immuno Research). Images were acquired with a MetaXpress Micro XL system (Molecular Devices) at 20x or 40x magnification.

### Biolayer interferometry (BLI)

To determine the binding affinity of purified Nbs to recombinant hCD4, biolayer interferometry (BLItz, ForteBio) was performed. First, CD4 was biotinylated by 3-fold molar excess of biotin-N-hydroxysuccinimide ester. CD4 was then immobilized at single-use high-precision streptavidin biosensors (SAX) according to manufacturer's protocols. For each Nb four association/dissociation runs were executed with concentrations appropriate for the affinities of the respective nanobodies (overall between 15.6 nM and 1 µM). As a reference run, PBS was used instead of Nb in the association step. As negative control the GFP-Nb (500 nM) was applied in the binding studies. Recorded sensograms were analyzed using the BLItzPro software and dissociation constants (K_{D}) were calculated based on global fits. For the epitope competition analysis, two consecutive application steps were performed, with a short dissociation period of 30 s after the first association.

### Affinity determination by flow cytometry

For cell-based affinity determination, HEK293-hCD4 were detached using enzyme-free cell dissociation buffer (Gibco) and resuspended in FACS buffer (PBS containing 5% FCS). For each staining condition 200,000 cells were incubated with suitable dilution series of CD4 nanobodies at 4°C for 30 min. Cells were washed two times and for detection Cy5 AffiniPure Goat Anti-Alpaca IgG, VHH domain (Jackson ImmunoResearch) was applied for 15 min. PBMCs (Department of Immunology/ ZKT Tübingen gGmbH) were freshly thawed and resuspended in FACS buffer. For each sample 200,000 cells were incubated with suitable concentrations of CD4 nanobodies coupled to CF568 in combination with 1:500 dilution of anti-CD3-FITC (BD Biosciences) at 4°C for 30 min. For control staining PE/Cy5-labeled anti-human CD4 antibody (RPA-T4, Biolegend) was used. After two washing steps, samples were resuspended in 200 µl FACS buffer and analyzed with a BD FACSMelody Cell Sorter. Final data analysis was performed via FIoJo10^{®} software.

### Sortase labeling of nanobodies

Sortase A pentamutant (eSrtA) in pET29 was a gift from David Liu (Addgene plasmid # 75144; http://n2t.net/addgene:75144; RRID:Addgene_75144) and was expressed and purified as previously described. CF568-coupled peptide H-Gly-Gly-Gly-Doa-Lys-NH₂ (sortase substrate) was custom-synthesized by Intavis AG. For the click-chemistry a peptide H-Gly-Gly-Gly-propyl-azide was synthesized. In brief, for sortase coupling 50 µM nanobody, 250 µM sortase peptide dissolved in sortase buffer (50 mM Tris, pH 7.5, and 150mM NaCl) and 10 µM sortase were mixed in coupling buffer (sortase buffer with 10 mM CaCl₂) and incubated for 4 h at 4°C. Uncoupled nanobody and sortase were depleted by IMAC. Unbound excess of unreacted sortase peptide was removed using Zeba Spin Desalting Columns (ThermoFisher Scientific, cat. #89890). Azide-coupled Nbs were labeled by SPAAC (strain-promoted azide-alkyne cycloaddition) click chemistry reaction with 2-fold molar excess of DBCO-Cy5.5 (Jena Biosciences) for 2 h at 25°C. Excess DBCO-Cy5.5 was subsequently removed by dialysis (GeBAflex-tube, 6-8 kDa, Scienova). Finally, to remove untagged nanobody, (side product of the sortase reaction), hydrophobic interaction chromatography was used (HIC, HiTrap Butyl-S FF, Cytiva). Binding of DBCO-Cy5.5-coupled Nb occurred in 50 mM H₂NaPO₄, 1.5 M (NH4)₂SO₄, pH7.2. Elution took place with 50 mM H₂NaPO₄, pH7.2. Dye-labeled protein fractions were analyzed by SDS-PAGE followed by fluorescent scanning on a Typhoon Trio (GE-Healthcare, CF568: excitation 532 nm, emission filter settings 580 nm BP 30; Cy5.5 excitation 633 nm, emission filter settings 670 nm BP 30; 546) and subsequent Coomassie staining. Identity and purity of final products were determined by LC-MS (CD4-Nbs-CF568, >60%; CD4-Nb1-Cy5.5, ~94%; CD4-Nb4-Cy5.5, ~99%; GBP-Cy5.5; ~94%, CD4-Nb1-3, ~99%; bivGFP-Nb, -99%).

### Hydrogen-Deuterium Exchange

### CD4 Deuteration Kinetics and Epitope Elucidation

CD4 (5 µL, 65.5 µM) was either incubated with PBS or CD4-specific Nbs (5 µL 0.9, 1.1 and 2.2 mg/ml in PBS) at 25°C for 10 min. Deuterium exchange of the preincubated nanobody-antigen complex was initiated by dilution with 90 µL PBS (150 mM NaCl, pH 7.4) prepared with D2O and incubation for 5 and 50 minutes respectively at 25°C. To ensure a minimum of 90% of complex formation, the molar ratio of antigen to Nbs was calculated according to Kochert et al. (Hydrogen-Deuterium Exchange Mass Spectrometry to Study Protein Complexes. Methods Mol Biol 1764, 153-171, doi:10.1007/978-1-4939-7759-8_10 (2018)) (using the affinity constants of 5.1 nM (CD4-Nb1), 6.5 nM (CD4-Nb2), 75.3 nM (CD4-Nb3) (pre-determined by BLI analysis). The final D2O concentration was 90%. After 5 and 50 min at 25°C, aliquots of 20 µL were taken and quenched by adding 20 µL ice-cold quenching solution (0.2 M TCEP with 1.5% formic acid and 4 M guanidine HCl in 100 mM ammonium formate solution pH 2.2) resulting in a final pH of 2.5. Quenched samples were immediately snap-frozen. The immobilized pepsin was prepared by adding 60 µl of 50% slurry (in ammonium formate solution pH 2.5) to a tube and dried by centrifugation at 1000 × g for 3 min at 0°C and discarding the supernatant. Before injection, aliquots were thawed and added to the dried pepsin beads. Proteolysis was performed for 2 min in a water ice bath followed by filtration using a 22 µm filter and centrifugation at 1000 × g for 30 s at 0°C. Samples were immediately injected into a LC-MS system. Undeuterated control samples were prepared under the same conditions using H2O instead of D2O. The same protocol was applied for the Nbs without addition of CD4 as well to create a list of peptic peptides. The HDX experiments of the CD4-Nb-complex were performed in triplicates. The back-exchange of the method as determined using a standard peptide mixture of 14 synthetic peptides was 24%.

### Chromatography and Mass Spectrometry

HDX samples were analyzed on a LC-MS system comprised of RSLC pumps (UltiMate 3000 RSLCnano, Thermo Fisher Scientific, Dreieich, Germany), a chilling device for chromatography (MéCour Temperature Control, Groveland, MA, USA) and a mass spectrometer Q Exactive (Thermo Fisher Scientific, Dreieich, Germany). The chilling device contained the LC column (ACQUITY BEH C18, 1.7 µm, 300 Å, 1 mm × 50 mm (Waters GmbH, Eschborn, Germany)), a cooling loop for HPLC solvents, a sample loop, and the injection valve and kept all components at 0°C. Samples were analyzed using a two-step 20 min linear gradient with a flow rate of 50 µl/min. Solvent A was 0.1% (v/v) formic acid and solvent B was 80% acetonitrile (v/v) with 0.1% formic acid (v/v). After 3 min desalting at 10% B, a 9 min linear gradient from 10 to 25% B was applied followed by an 8 min linear gradient from 25 to 68.8%. Experiments were performed using a Q Exactive (Thermo Fisher Scientific, Dreieich, Germany) with 70,000 resolutions instrument configurations as follows: sheath gas flow rate of 25; aux gas flow rate of 5; S-lens RF level of 50, spray voltage of 3.5 kV and a capillary temperature of 300°C.

### HDX Data Analysis

A peptic peptide list containing peptide sequence, retention time and charge state was generated in a preliminary LC-MS/MS experiment. The peptides were identified by exact mass and their fragment ion spectrum using protein database searches by Proteome Discoverer v2.1.0.81 (Thermo Fisher Scientific, Dreieich, Germany) and implemented SEQUEST HT search engine. The protein database contained the CD4 and the pepsin sequences. Precursor and fragments mass tolerance were set to 6 ppm and 0.05 Da, respectively. No enzyme selectivity was applied, however, identified peptides were manually evaluated to exclude peptides originated through cleavage after arginine, histidine, lysine, proline and the residue after proline ⁷⁷. FDR was estimated using q-values calculated by Perculator and only peptides with high-confidence identification (q-value ≤ 0.01) were included to the list. Peptides with overlapping mass, retention time and charge in Nb and antigen digest, were manually removed. The deuterated samples were recorded in MS mode only and the generated peptide list was imported into HDExaminer v2.5.0 (Sierra Analytics, Modesto, CA, USA). Deuterium uptake was calculated using the increase of the centroid mass of the deuterated peptides. HDX could be followed for 87% of the CD4 amino acid sequence. The calculated percentage deuterium uptake of each peptide between CD4-Nb and CD4-only were compared. Any peptide with uptake reduction of 5% or greater upon Nb binding was considered as protected.

### Endotoxin determination and removal

The concentration of bacterial endotoxins was determined with Pierce LAL Chromogenic Endotoxin Quantitation Kit (Thermo Fisher Scientific) and removal occurred using EndoTrap HD 1 ml (Lionex) according to the manufacturers' protocols.

### PBMC isolation, cell freezing, and thawing

Fresh blood, buffy coats, or mononuclear blood cell concentrates were obtained from healthy volunteers at the Department of Immunology or from the ZKT Tübingen gGmbH. Participants gave informed consent and the studies were approved by the ethical review committee of the University of Tübingen, projects 156/2012B01 and 713/2018BO2). Blood products were diluted with PBS 1x (homemade, 10x Lonza, Switzerland) and peripheral blood mononuclear cells (PBMCs) were isolated by density gradient centrifugation with Biocoll separation solution (Biochrom, Germany). PBMCs were washed twice with PBS 1x, counted with a NC-250 cell counter (Chemometec, Denmark), and resuspended in heat-inactivated (h.i.) fetal bovine serum (Capricorn Scientific, Germany) containing 10% DMSO (Merck, Germany). Cells were immediately transferred into a -80°C freezer in a freezing container (Mr. Frosty; Thermo Fisher Scientific, USA). After at least 24 hours, frozen cells were transferred into a liquid nitrogen tank and were kept frozen until use. For the experiments, cells were thawed in IMDM (+L-Glutamin +25mM HEPES; Life Technologies, USA) supplemented with 2.5% h.i. human serum (HS; PanBiotech, Germany), 1x Penicillin/Streptomycin (P/S; Sigma-Aldrich, Germany), and 50 µm β-Mercaptoethanol (β-ME; Merck, Germany), washed once, counted and used for downstream assays.

### Synthetic peptides

The following HLA-class II peptides were used for the stimulations: MHC class II pool (HCMVA pp65 aa 109-123 MSIYVYALPLKMLNI (SEQ ID No. 13), HCMV pp65 aa 366-382 HPTFTSQYRIQGKLEYR (SEQ ID No. 14), EBVB9 EBNA2 aa 276-290 PRSPTVFYNIPPMPL (SEQ ID No. 15), EBVB9 EBNA1 aa 514-527 KTSLYNLRRGTALA (SEQ ID No. 16), EBV BXLF2 aa 126-140 LEKQLFYYIGTMLPNTRPHS (SEQ ID No. 17), EBV BRLF1 aa 119-133 DRFFIQAPSNRVMIP (SEQ ID No. 18), EBVB9 EBNA3 aa 381-395 PIFIRRLHRLLLMRA (SEQ ID No. 19), EBVB9 GP350 aa 167-181 STNITAVVRAQGLDV (SEQ ID No. 20), IABAN HEMA aa 306-318 PKYVKQNTLKLAT (SEQ ID No. 21) or CMVpp65 aa 510-524 YQEFFWDANDIYRIF (SEQ ID No. 22). All peptides were synthesized and dissolved in water 10% DMSO.

### Stimulation and cultivation of PBMCs

PBMCs from donors previously screened for *ex vivo* CD4⁺ T cell reactivities against MHC-class II peptides were thawed and rested in T cell medium (TCM; IMDM + 1x P/S + 50µM β-ME + 10% h.i. HS) containing 1µg/mL DNase I (Sigma-Aldrich, Germany) at a concentration of 2-3×10⁶ cells/mL for 3h at 37°C and 7.5% CO₂. After resting, cells were washed once, counted and up to 1×10⁸ cells were labeled with 1.5-2 µM Carboxyfluorescein succinimidyl ester (CFSE; BioLegend, USA) in 1 mL PBS 1X for 20 min according to the manufacturer's protocol. The cells were washed twice in medium containing 10% FBS after CFSE labeling and incubated with 5 µM, 0.5 µM, or 0.05 µM of CD4-Nb1, CD4-Nb4 or a control Nb for 1h at 37°C in serum-free IMDM medium. Concentrations and duration were chosen to mimic the expected approximate concentration and serum retention time during clinical application. After incubation, cells were washed twice, counted and each condition was separated into three parts and seeded in a 48-well cell culture plate (1.6-2.5×10⁶ cells/well in triplicates). Cells were stimulated with either 10 µg/ml PHA-L (Sigma-Aldrich), 5 µg/mL MHC class-II peptide(s) or left unstimulated, and cultured at 37°C and 7.5% CO₂. 2 ng/mL recombinant human IL-2 (R&D, USA) were added on days 3, 5, and 7. One third of the culture on day 4, one half of the culture on day 6 and day 8, and the remaining cells on day 12 were harvested, counted and stained for flow cytometry analyses. For donor 1, the proliferation/activation status and cytokine production were analyzed in two different experiments, whereas for donors 2 and 3, cells from a single experiment were used for the three assays.

### Assessment of T cell proliferation and activation

Cells from days 4, 6, and 8 were transferred into a 96-well round-bottom plate and washed twice with FACS buffer (PBS + 0.02% sodium azide (Roth, Germany) +2 mM EDTA (Sigma-Aldrich, Germany) +2% h.i. FBS). Extracellular staining was performed with CD4 APC-Cy7 (RPA-T4, BD Biosciences), CD8 BV605 (RPA-T8, BioLegend), the dead cell marker Zombie Aqua (BioLegend), CD25 PE-Cy7 (BC96, BioLegend), CD69 PE (FN50, BD Biosciences) and incubated for 20 min at 4°C. All antibodies were used at pre-tested optimal concentrations. Cells were washed twice with FACS buffer. Approx. 500.000 cells were acquired on the same day using a LSRFortessaTM SORP (BD Biosciences, USA) equipped with the DIVA Software (Version 6, BD Biosciences, USA). The percentage of proliferating CD4⁺ cells was determined by assessment of CFSE negative cells, activation by the percentage of CD69⁺ or CD25⁺.

### Assessment of T cell function by intracellular cytokine staining

On day 12, the MHC class II peptide(s)-stimulated and cultured cells were transferred into a 96-well round-bottom plate (0.5 to 1x 10⁶ cells/well) and restimulated using 10 µg/ml of the same peptide(s), 10 µg/ml Staphylococcus enterotoxin B (SEB, Sigma-Aldrich; positive control), or 10% DMSO (negative control). Protein transport inhibitors Brefeldin A (10 µg/ml, Sigma-Aldrich) and Golgi Stop (BD Biosciences) were added at the same time as the stimuli. After 14 h stimulation at 37°C and 7.5% CO₂, cells were stained extracellularly with the fluorescently labeled antibodies CD4 APC-Cy7, CD8 BV605, and Zombie Aqua and incubated for 20 min at 4°C. After, cells were washed once, then fixed and permeabilized using the BD Cytofix/Cytoperm solution (BD Biosciences) according to the manufacturer's instructions, stained intracellularly with TNF Pacific Blue (Mab11), IL-2 PE-Cy7 (MQ1-17H12), IFN-γ AlexaFluor 700 (4S.B7) and CD154 APC (2431) antibodies (all BioLegend) ⁷⁹ and washed twice. Approx. 500.000 cells were acquired on the same day using a LSRFortessaTM SORP (BD Biosciences, USA), equipped with the DIVA Software (Version 6, BD Biosciences, USA). All flow cytometry analyses were performed with FlowJo version 10.6.2, gating strategies are shown in Fig. 9. Statistical analyses were performed with GraphPad Prism version 9.0.0.

### Full blood stimulation and cytokine release assay

100 µl of lithium-heparin blood was incubated for 1h at 37°C and 7.5% CO₂. After, the blood was stimulated with 5 µM Nb (CD4-Nb1, CD4-Nb4 or control Nb), with 100 ng/mL LPS (Invivogen, USA), or with 2 µg/mL PHA-L in a final volume of 250 µl (serum-free IMDM medium), or left unstimulated for 24 h at 37°C and 7.5% CO₂. After two centrifugations, supernatant was collected without transferring erythrocytes. The supernatants were frozen at -80°C until cytokine measurements. Levels of IL-1β, IL-1Ra, IL-4, IL-6, IL-8, IL-10, IL-12p70, IL-13, GM-CSF, IFNγ, MCP-1, MIP-1β, TNFα and VEGF were determined using a set of "in house developed" Luminex-based sandwich immunoassays each consisting of commercially available capture and detection antibodies and calibrator proteins. All assays were thoroughly validated ahead of the study with respect to accuracy, precision, parallelism, robustness, specificity and sensitivity. Samples were diluted at least 1:4 or higher. After incubation of the pre-diluted samples or calibrator protein with the capture coated microspheres, beads were washed and incubated with biotinylated detection antibodies. Streptavidin-phycoerythrin was added after an additional washing step for visualization. For control purposes, calibrators and quality control samples were included on each microtiter plate. All measurements were performed on a Luminex FlexMap^{®} 3D analyzer system, using Luminex xPONENT^{®} 4.2 software (Luminex, Austin, TX, USA). For data analysis MasterPlex QT, version 5.0 was employed. Standard curve and quality control samples were evaluated according to internal criteria adapted to the Westgard Rules to ensure proper assay performance.

### Analysis of cross-species reactivity binding to mouse CD4⁺ cells by flow cytometry

Mouse CD4⁺ cells were isolated from spleen and lymph nodes of C57BL/6N mice by positive selection over CD4 magnetic microbeads (Miltenyi Biotech, Bergisch Gladbach, Germany). Human CD4⁺ cells were extracted from blood using StraightFrom^{®} Whole Blood CD4 MicroBeads (Miltenyi Biotech). Single cell suspensions were incubated with 0.75 µg/ml of CD4-Nbs-Cy5.5 (~47 - 60 nM) or GFP-Nb-Cy5.5 (~51 nM) in 1% foetal calf serum/PBS at 4°C for 20 min and subsequently analyzed on an LSR-II cytometer (BD biosciences).

### Optical Imaging of CD4-expressing HPB-ALL tumors

Human T cell leukemia HPB-ALL cells (German Collection of Microorganisms and Cell Cultures GmbH, DSMZ, Braunschweig, Germany) were cultured in RPMI-1640 supplemented with 10% foetal calf serum and 1% penicillin/streptomycin. 10⁷ HPB-ALL cells were injected s.c. in the right upper flank of 7-week-old NOD SCID gamma mice (NSG, NOD.Cg-*Prkdc^{scid} II2rg^{tm1Wjl}*/SzJ, Charles River Laboratories, Sulzfeld, Germany) and tumor growth was monitored for 2-3 weeks. When tumors reached a diameter ~7 mm, 5 µg of CD4-Nbs-Cy5.5 or control Nb (GFP-Nb-Cy5.5) were administered into the tail vein of 2 mice each. Optical imaging (OI) was performed repetitively in short-term isoflurane anesthesia over a period of 24 h using the IVIS Spectrum In Vivo Imaging System (PerkinElmer, Waltham, MA, USA). Four days after the first Nb administration, the CD4-Nbs-Cy5.5 groups received the GFP-Nb-Cy5.5 (and vice versa) and tumor biodistribution was analyzed identically by OI over 24 h. After the last imaging time point, animals were sacrificed and tumors were explanted for *ex vivo* OI analysis. Data were analyzed with Living Image 4.4 software (PerkinElmer). The fluorescence intensities were quantified by drawing regions of interest around the tumor borders and were expressed as average radiant efficiency (photons/s)/(*µ*W/cm²) subtracted by the background fluorescence signal before Nb injection. All experiments were performed according to the German Animal Protection Law and were approved by the local authorities (Regierungspräsidium Tübingen).

### Analyses and Statistics

Data analysis of the flow cytometry data was performed with the FlowJo Software Version 10.6.2 (FlowJo LLT, Ashland, Oregan, USA) and graph preparation and statistical analysis was performed using the GraphPad Prism Software (Version 8.3.0 or higher).

### Results

### Generation of high-affinity CD4 nanobodies

To generate Nbs directed against human CD4 (hCD4), an alpaca (*Vicugna pacos*) was immunized with the recombinant extracellular portion of hCD4 following an 87-day immunization protocol. Subsequently, a Nb phagemid library was generated comprising ~4 × 10⁷ clones that represent the full repertoire of variable heavy chains of heavy-chain antibodies (VHHs or Nbs) of the animal. Phage display was performed using either passively adsorbed purified hCD4 or CHO cells stably expressing full-length human CD4 (CHO-hCD4 cell line). Following two cycles of phage display for each condition, a total of 612 individual clones were analyzed by whole-cell phage ELISA and identified 21 positive binders. Sequence analysis revealed 13 unique Nbs representing five different B-cell lineages according to their complementarity determining regions (CDR) 3 (Fig. 1 A). One representative Nb of each lineage, termed CD4-Nb1 - CD4-Nb5, was expressed in bacteria (E.coli) and isolated with high purity using immobilized metal ion affinity chromatography (IMAC) followed by size exclusion chromatography (SEC) (Figure 1 B). To test whether selected Nbs are capable of binding to full-length hCD4 localized at the plasma membrane of mammalian cells, live-cell staining of CHO-hCD4 cells was performed (Figure 1 C). Executed at 4°C, images show staining of the plasma membrane, whereas at 37°C, the fluorescent signal localized throughout the cell body, presumably a consequence of endocytotic uptake of receptor-bound Nbs. CHO wt cells were not stained by any of the five CD4-Nbs at both temperatures (data not shown). CD4-Nb1 and CD4-Nb3, both identified by whole-cell panning, displayed strong staining of CHO-hCD4 cells. Of the Nbs derived from panning with recombinant hCD4, CD4-Nb2 also showed strong cellular staining, whereas staining with CD4-Nb4 revealed weak signals and CD4-Nb5 showed no staining under these conditions and was consequently excluded from further analyses (Figure 1 C).

To quantitatively assess affinities, biolayer interferometry (BLI) measuring serial dilutions of Nbs were performed on the biotinylated extracellular domain of hCD4 immobilized at the sensor tip. For CD4-Nb1 and CD4-Nb2, determined KD values were in the low nanomolar range with ~5 and ~7 nM, respectively, while CD4-Nb3 and CD4-Nb4 displayed lower affinities of 75 nM and 135 nM, respectively (Figure 1 D, Table 1, Fig. 6A). In addition, corresponding EC50 values were determined with full-length plasma membrane-located hCD4 on HEK293-hCD4 cells by flow cytometry. In accordance with cellular staining and biochemically determined affinities these values revealed a strong functional binding for CD4-Nb1 and CD4-Nb2 with EC50 values in the subnanomolar range (~0.7 nM), whereas CD4-Nb3 and CD4-Nb4 displayed substantially lower cellular affinities (Figure 1 E, Table 1, Fig. 6A). Here, exemplarily, four CD4-Nbs were generated that bind isolated as well as cell-resident hCD4. While CD4-Nb4 appeared less affine, CD4-Nb1 and CD4-Nb2 displayed high affinities in the low nanomolar range.

### Domain mapping

Next, enzymatic coupling was applied using sortase A for site-directed functionalization of CD4-Nbs. Thereby, peptides conjugated to a single fluorophore were linked to the C-terminus of CD4-Nbs yielding a defined labeling ratio of 1:1 42. Live-cell immunofluorescence imaging employing CD4-Nbs sortase-coupled to the fluorescent dye CF568 showed that all CD4-Nbs retained their capability of binding to cell-resident hCD4 of CHO-hCD4 cells (data not shown). In order to localize the binding site of the selected CD4-Nbs, domain-deletion mutants of hCD4 were generated. Expression and surface localization of these mutants was confirmed by staining with antibody RPA-T4 binding to domain D1 of CD4. For mutants lacking domain D1, an N-terminal BC2 tag 43 was introduced to allow for live-cell surface detection with a fluorescently labeled bivBC2-Nb 42 (data not shown). Transiently expressed domain-deletion mutants were tested for binding of CF568-labeled CD4-Nbs by live-cell immunofluorescence imaging. As control a non-specific fluorescently labeled GFP-binding Nb (GFP-Nb) was added (Figure 2 A, and data not shown).

Based on these results binding of CD4-Nb1 and CD4-Nb3 was allocated to domain D1, whereas CD4-Nb2 and CD4-Nb4 bind to domain D3 and/or D4 of hCD4. No binding of the GFP-Nb was detected. To further examine combinatorial binding of the different CD4-Nbs, epitope binning analysis was performed by BLI. Recombinant full-length hCD4 was immobilized at the sensor tip and combinations of CD4-Nbs were allowed to bind consecutively (Fig. 7). Unsurprisingly, CD4-Nbs binding to different domains, displayed combinatorial binding. Interestingly a simultaneous binding was detected for the combination of CD4-Nb1 and CD4-Nb3, both targeting domain D1 of hCD4 indicating that both CD4-Nbs bind to different epitopes within domain D1. In contrast, simultaneous binding for CD4-Nb2 and CD4-Nb4 was not observed, suggesting the possibility of close-by or overlapping epitopes at domains D3/4 for the latter Nb pair. To further support these findings and more precisely localize the respective binding sites, a hydrogen-deuterium-exchange (HDX) mass spectrometry analysis of hCD4 bound to CD4-Nb1, CD4-Nb2 or CD4-Nb3 (Figure 2 B-E) was conducted. Due to its low affinity, CD4-Nb4 was not subjected to HDX-MS analysis. In accordance with data obtained from immunofluorescence staining of the domain-deletion mutants and the combinatorial binding analysis, binding of CD4-Nb1 and CD4-Nb3 protected sequences of domain D1 from HDX, whereas CD4-Nb2 protected sequences of domain D3 and D4 of hCD4 (Figure 2 B).

Results obtained for binding of CD4-Nb1 (Figure 2 C) are similar to those of CD4-Nb3 (Figure 2 D) in that binding of either Nb decreases exchange in the sequence ranging from amino acid residue (aa) T17 through N73, albeit with a differing extent of protection at individual sequence sections. For CD4-Nb1 the greatest protection from HDX was observed for sequence ranging from aa K35 - L44 corresponding to β strand C*'* and Cʺ of the immunoglobulin fold of domain D1 and residues aa K46 - K75, comprising β strands D and E. In contrast, binding of CD4-Nb3 confers only a minor reduction in HDX within the latter sequence, but additionally protects sequence aa C84 - E91, which correspond to β strands G and F and their intermediate loop. Binding of CD4-Nb2 protects sequences aa W214 - F229 (β strands C and C') and aa K239 - L259 (β strands Cʺ-E), and to a minor extend sequence aa R293 - L296 as part of β strands A of domain D4 (Figure 2 E). In summary, CD4-Nb1 and CD4-Nb3 are able to simultaneously bind their epitopes located in domain D1 of hCD4, whereas the epitope of CD4-Nb2 is mainly located at domain D3. Based on the inability of CD4-Nb4 to bind hCD4 simultaneously with CD4-Nb2 it was assessed that the latter two Nbs possess close-by or overlapping epitopes.

### Binding of CD4-Nbs to human PBMCs

Having demonstrated that all selected Nbs bind to recombinant and exogenously overexpressed cellular hCD4, next, their capability and specificity of binding to physiologically relevant levels of endogenous CD4 on human PBMCs were determined. Human peripheral blood mononuclear cells (PBMCs) from three donors were co-stained with CD4-Nbs1-4 coupled to CF568 (100 nM for high-affine CD4-Nb1 and CD4-Nb2; 1000 nM for low-affine CD4-Nb3 and CD4-Nb4) in combination with an anti-CD3 antibody and analyzed the percentage of double-positive cells (CD3+CD4+) by flow cytometry (Figure 3 A, Fig. 8). Compared to staining with an anti-CD4 antibody as positive control, all CD4-Nbs stained a similar percentage of cells for all tested donors, while the non-specific GFP-Nb yielded a negligible percentage of double-positive cells even at the highest concentration (1000 nM) (Figure 3 B, Table 2).

### Impact of CD4-Nbs on activation, proliferation and cytokine release of CD4+ T and immune cells

Towards the envisioned application as clinical imaging tracer, basic issues associated with CD4-Nbs regarding their influence on the activation, proliferation and cytokine release of CD4+ T cells were assessed. First, to exclude adverse effects of bacterial endotoxins present in the CD4-Nbs1-4 preparations, endotoxins were removed by depletion chromatography to yield FDA acceptable endotoxin levels of <0.25 EU per mg for CD4-Nb1, CD4-Nb4 and the non-specific GFP-Nb.

Consequently, these experimental studies were continued with CD4-Nb1, CD4-Nb4 and GFP-Nb as control. In brief, carboxyfluorescein succinimidyl ester (CFSE)-labeled human PBMCs from three pre-selected healthy donors were pre-treated with CD4-Nbs or a control Nb at concentrations between 0.05 µM - 5 µM for 1 h at 37°C, mimicking the expected approximate concentration and serum retention time during clinical application. Cells were then washed to remove Nbs and stimulated with an antigenic (cognate MHCII peptides) or a non-antigenic stimulus (phytohaemagglutinin, PHA-L) and analyzed after 4, 6 and 8 days by flow cytometry with the gating strategy shown in Fig. 9A. According to the highly similar CFSE intensity profiles observed, the total number of cell divisions was not affected by the different Nb treatments (exemplarily shown for one of three donors on day 6, Fig. 9 A). For samples of the same donor and time point, no substantial differences in the percentage of proliferated cells were observed between no Nb addition and individual Nb treatments. Although varying between donors, for both stimuli, the average percentage of proliferated cells increased over time without marked differences between conditions (Figure 4 A). As quantitative measure of T cell activation, also the cell surface induction of a very early activation marker (CD69) and of the IL-2 receptor α chain (CD25) on CD4+ T cells was determined (Figure 4 B). Among samples of the same donor and stimulation, highly similar activation profiles for all Nb treatments were found. While the percentage of CD25+ cells steadily increased over time for MHCII peptide stimulation, for PHA-stimulated condition the percentage of positive cells was similarly high at all times of analysis. Importantly, regardless of the differences between donors, at no point in the analysis did individual Nb treatments from the same donor result in significant differences in the percentage of CD4+CD25+ or CD4+CD69+ cells for both stimulations.

Next, cytokine expression of CD4+ T cells were analyzed by intracellular cytokine staining after restimulation with cognate MHCII peptides. The corresponding gating strategy is shown in Fig. 9B. Samples of the same donor treated with different Nbs had highly similar percentages of cytokine (TNF, IFN-γ or IL-2) or activation marker (CD154)-positive cells without stimulation and upon stimulation with MHCII peptides (Figure 4 C). Overall, exposure to CD4-Nbs did not affect proliferation, activation or cytokine production of CD4+ T cells. In addition, potential effects of CD4-Nbs on the release of cytokines from full-blood samples of three further donors were analyzed. Upon stimulation with lipopolysaccharide (LPS) or PHA L, the serum concentrations were determined with a panel of pro- and anti-inflammatory cytokines (**Fig. 20B**). Although there were significant differences between donors for some cytokines, the presence of CD4-Nbs did not result in significant differences in either stimulated or unstimulated samples (Fig. 10).

### In vivo optical imaging of CD4-Nbs

For optical *in vivo* imaging, CD4-Nbs were labeled with the fluorophore Cy5.5 (CD4-Nb-Cy5.5) by sortase-mediated attachment of an azide group followed by click-chemistry addition of DBCO-Cy5.5. First, potential cross-reactivity of the four Cy5.5-labeled CD4-Nbs to murine CD4+ lymphocytes was tested. Notably, flow cytometric analysis showed that none of the selected CD4-Nbs was able to bind murine CD4+ cells, suggesting exclusive binding to human CD4. Moreover, low-affine binding CD4-Nb4 bound neither mouse nor human CD4+ cells at the concentration used here (0.75 µg/ml, -49 nM) (Fig. 11). CD4-Nb1 as and CD4-Nb4 were further analyzed for their *in vivo* target specificity and dynamic distribution using a murine xenograft model.

To establish human CD4+ expressing tumors, NSG mice were inoculated subcutaneously with CD4+ T cell leukemia HPB-ALL cells 44. After 2 ― 3 weeks mice bearing HPB-ALL xenografts were injected with 5 µg of CD4-Nb1-Cy5.5, CD4-Nb4-Cy5.5, or a control Nb (GFP-Nb-Cy5.5) and optically imaged in intervals over the course of 24 h (Figure 5 A, Fig. 12 A). The Cy5.5 signal intensity (SI) of control Nb peaked within 10 ― 20 minutes and rapidly declined thereafter to the half and quarter of maximum level at 2 h and 24 h, respectively (Figure 5 B, Fig. 12 B). While the SI low-affinity CD4-Nb4-Cy5.5 did not exceed the SI of the control Nb at any time (Fig. 12 B), CD4-Nb1-Cy5.5 reached the maximum SI within the HPB-ALL ~1.8-fold above the control Nb, at 30 min and slowly declined to ~90% and ~80% of maximum after 2 and 4 hours, respectively (Figure 5 B). Based on the differences in the SI of the HPB-ALL xenografts of experimental mice between CD4-Nb1-Cy5.5 and GFP-Nb-Cy5.5 as a basis, the optimal target accumulation and specificity was observed between 60 and 120 min post injection (Figure 5 B). After 24 h, mice were euthanized, and presence of fluorophore-labeled CD4 Nbs within the explanted tumors was analyzed by OI (Figure 5 C, Fig. 12 C). Compared to control, tumors from mice injected with CD4-Nb1-Cy5.5 had ~4-fold higher Cy5.5 SI, indicating a good signal-to-noise ratio for the Nb-derived fluorescently labeled immunoprobe.

To confirm CD4-specific targeting of CD4-Nb1 within the xenograft, ex *vivo* immunofluorescence of HPB-ALL tumors at 2 hours and 24 hours post injection was performed (Fig. 13). At the early time point, when the *in vivo* OI signal peaked, CD4-Nb1 was widely distributed throughout the whole tumor whereas no Cy5.5 signal was detected in the GFP-Nb-injected mice (Fig. 13 A, B). Semiquantitative analysis at the single-cell level revealed intense CD4-Nb1 binding at the surface of HBP-ALL cells that correlated with the CD4 antibody signal and internalization of CD4-Nb1 in some cells (Fig. 13 C). Upon administration of unrelated GFP-Nb, no Nb binding was observed (Fig. 13 D). 24 hours post injection regions of strongly internalized CD4-Nb1 (Fig. 13 E, G) were observed, but also regions showing a low residual CD4-Nb1 uptake (Fig. 13 E, H).

The above data strongly indicate that high-affinity binding CD4-Nb1 is suitable for visualizing CD4⁺ cells *in vivo* by non-invasive optical imaging.

### Summarizing discussion

In the invention presented herein, CD4 Nbs were developed, which can be used - as biologically inert nanobodies - e.g. as novel immunoprobes for visualization and monitoring of human CD4+ T cells in various biomedical research applications, focusing on non-invasive *in vivo* imaging. Beyond application as a diagnostic agent, CD4-Nbs offer various therapeutic opportunities. Considering the cellular function of CD4, a detailed epitope characterization is of particular interest as Nbs might modulate endogenous CD4 depending on their recognized epitope, e.g. by affecting transient interactions e.g. as they occur between CD4 and the MHCII complex or through blockade of ligand binding. While the MHC-II binding is located to the domain D1 of the CD4 antigen, T cell activation is abrogated when TCR and CD4 colocalization is blocked via domain.

From the data generated with the present invention, it can be concluded that the newly generated Nbs are biologically inert and thus beneficial compared to full length antibodies or other antibody fragments. Also, the provided data prove that the newly generated CD4-Nbs are suitable probes for *in vivo* imaging. Considering that the selected CD4 Nbs bind exclusively to human CD4 but not to the homolog in mice, a xenograft mouse model was used in which a CD4+ T leukemia tumor model was implemented based on human derived HBP-ALL cells. Using this model, the specific enrichment of the high affine CD4-Nb1 on HBP-ALL derived xenografts could be monitored by optical *in vivo* imaging.

### 2. Targeting of CD4+ T cells by nanobody-modified AAV2 gene therapy vectors

### Material and methods

### Plasmid construcution

The plasmid pAAV2/2 (Addgene #104963) encoding wildtype AAV2 rep/cap genes served as a starting point for capsid optimization and blinding. Amino acid substitutions for capsid optimization (Y444F, T491V, Y500F and Y730F), as well as disruption (blinding) of the primary natural AAV2 heparan sulfate proteoglycan (HSPG) binding site (R585A and R588A) have been described before. Genetic modification of the respective nucleotides was conducted by site-directed mutagenesis (SDM) PCR using DNA oligonucleotides in a back-to-back orientation (oligonucleotides oMH19 - oMH27;Fig. 22) and Q5 DNA polymerase (New England Biolabs); PCR program: initial denaturation 2 min 8°C, 18 cycles denaturation 10" 98°C, annealing 30" 55°C, elongation 6 min 72°C, and final elongation 3 min 72°C. PCR was followed by a "one pot" reaction including a Dpnl (Thermo Fisher Scientific) digest, 5' end phosphorylation with T4 Polynucleotide Kinase (New England Biolabs) and T4 DNA ligation (Thermo Fisher Scientific). After transformation into *E. coli* and plasmid isolation, the introduced nucleotide changes were verified by Sanger sequencing. Sequential rounds of SDM reactions yielded the construct pAAV2/2-o, encoding AAV2 capsid proteins with all four optimizations, as well as pAAV2/2-bo, encoding all four optimizations plus two HSPG-blinded amino acid substitutions.

AAV2 cap expression yields three capsid proteins, VP1-3: VP1 is translated from unspliced mRNA, whereas V2 and V3 are translation products from spliced mRNA. In order to achieve only VP1 expression, the major splice acceptor (SA) site was mutated. As described above, SDM PCR (oligonucleotides oMH47 and oMH48; see Fig. 22) with amplicon ligation and subsequent construct verification was performed, yielding the construct pAAV2/2-bo-SA. In contrast, after mutation of the AAV2 cap start codon (sc), the nascent construct allows only for VP2 and VP3 expression. Analogous to the protocol above, pAAV2/2-bo-sc was generated using oligonucleotides oMH45 and oMH46 (see Fig. 22).

### Generation of VP1-modified nanobody constructs

Nanobody and linker sequences were genetically inserted within the GH2/GH3 surface loop region of VP1. To achieve this a Notl recognition site at the respective location within cap was introduced into pAAV2/2-bo-SA (SDM PCR with oMH67 and oMH68). In parallel to the subsequent ligation reaction, the 5' linker sequence was introduced by a pair of annealed oligonucleotides (oMH69 and oMH70; see Fig. 22), yielding pAAV2/2-bo-SA-target-vector.

Nanobody and the 3' linker sequences were subsequently introduced by Gibson assembly following the manufacturer's instructions (Gibson Assembly Master Mix, NEB), using the NotI/XcmI linearized pAAV2/2-bo-SA-target-vector fragment and a single PCR fragment comprising the monovalent nanobody, 3' linker and cap sequences were ligated to the cap internal Xcml restriction site (insert fragment). To generate this insert fragment, two single PCR amplicons were fused by overlap extension PCR. First, nanobody sequences were PCR amplified from the above mentioned plasmids, using PCR oligos containing 5' homology to the target vector (forward oligo) as well as the 3' linker sequence plus 3' homology to the target vector (reverse oligo)(oligos for each nanobody listed in see Fig. 22). The second PCR fragment spanning the 3' linker sequence to the Xcml site within cap was generated using oligos oMH85/86.

The resulting VP1-modified nanobody constructs (VP1-CD4 -Nb1, - Nb1a, -Nb3, -Nb4 and PepNb) were verified by sequencing. The amino acid sequence of the final constructs is provided in see Fig. 21.

### Generation of VP2-modified nanobody constructs

First, the optimized and blinded VP2 open reading frame was PCR-amplified with DNA oligonucleotides introducing flanking XbaI and BamHI restriction sites from AAV2/2-bo cDNA (oligonucleotides: oMH16 and oMH18; see Fig. 22). After XbaI/BamHI amplicon digestion, the respective fragment was ligated into the XbaI/BamHI linearized pBC12/CMV vector backbone, resulting in construct pBC12-CMVint-VP2. Next, nanobody sequences were genetically fused via a linker sequence to the 5' end of the VP2 open reading frame in order to achieve expression of amino-terminal nanobody-linker-VP2 fusion peptides. To this end, a three fragment ligation strategy was applied comprising the HindIII/XbaI linearized pBC12-CMVint-VP2 vector fragment, the linker fragment consisting of the annealed oligonucleotides oMH33 and oMH34 with XbaI/XhoI-compatible ends, and the nanobody PCR insert fragment with XhoI/HindIII compatible ends (nanobody-specific oligonucleotides used for PCR amplification are listed in Fig. 22). All final constructs (VP2-CD4-Nb1, -Nb1a, -Nb3, -Nb4, -Nb5 and PepNb) were verified by Sanger sequencing. The amino acid sequences of the final constructs are provided in Fig. 21.

### Generation of VP1-modified bivalent nanobody constructs

Tandem VP1-biCD4-Nb1 and control VP1-biPepNB constructs were generated from synthesized DNA fragments (GeneArt, Sigma-Aldrich) containing Notl/Xcml restriction sites, linker sequences flanking each nanobody and VP1 sequences with optimized and blind modifications. Both GeneArt synthesis plasmids and pAAV2/2-bo-SA-target vector were digested with Notl/Xcml and the bivalent nanobody insert was ligated into the VP1-vector, resulting in pAAV2/-bo-SA-VP1-biCD4 and -biPepNB. Final constructs were verified by DNA sequencing. The amino acid sequence is provided in Fig. 21.

### Cell culture

All cell lines used in this study were described before and cultivation was performed under standard conditions in humidified atmosphere at 37°C and 5% CO₂. Adherent cell lines HEK 293T (ATCC: CRL-3216), HeLa (ATCC: CCL-2) and HeLa TZMbl (NIBSC: ARP5011) were passaged using 0.05% trypsin-EDTA (Pan Biotech) and cultured in DMEM growth medium (Pan Biotech) supplemented with 10% foetal calf serum (Biochrom), 2 mM L-glutamine and 50 U/ml penicillin/streptomycin antibiotics (Biochrom). Suspension T cell lines SupT1 (ATCC: CRL-1942) and Jurkat 1G5 (NIBSC: 5010) were cultivated in RPMI 1640 growth medium (Biozyme Scientific) supplemented with 10% foetal calf serum (Pan Biotec) and 50 U/ml penicillin-streptomycin.

Peripheral blood mononuclear cells (PBMC) were isolated from buffy coats of healthy donors. Isolation of CD4+ T cells from buffy coats was carried out using the RoboSep negative selection human CD4+ T cell enrichment kit in conjunction with a RoboSep automated cell separator (Stemcell Technologies) according to the manufacturer's instructions. Isolated cells were cultivated in RPMI 1640 (Biozyme Scientific) supplemented with 10% FCS, with 100 U/ml penicillin-streptomycin, 2 mM L-glutamine and 500 U/ml recombinant human IL-2 (Sigma-Aldrich).

### AAV particle production, purification and quantification

AAV production in HEK293T cells via polyethylenimin (PEI; Sigma-Aldrich) transfection was performed as described before. Briefly, 1 day post transfection 4×10⁶ cells were seeded per 10 cm dish. Transfection was conducted with a 4:1 PEI:DNA ratio in Opti-MEM (Life Technology) with a total of 10.5 µg plasmid DNA per dish (i.e. 42 µg PEI:10.5 µg DNA). The PEI transfection protocol was conducted following the manufacturer's instructions in a final volume of 6 mL per dish. In all co-transfection reactions the helper plasmid pAdDeltaF6 (Addgene #112867) and the AAV2 vector genome pscAAV-CAG-GFP (Addgene #83279) encoding the GFP transgene under a constitutive CAG promoter were included. Further plasmids were added to the mix as required (opt: pAAV2/2-o; opt-blind: pAAV2/2-bo; VP1-nanobody: pAAV2/2-bo-sc + pAAV2/2-bo-SA-VP1-NbX; VP2-nanobody: pAAV2/2-bo + pBC12-CMVint-VP2-NbX). All plasmids were co-transfected in equimolar quantities. Thirty hours post transfection, 6 mL of fresh medium was added to the cultures. Three days post transfection, cells were scraped off the dish and combined with the supernatant. After centrifugation (15 min, 2,800 × g), supernatant was discarded, the cell pellet resuspended in 800 µL lysis buffer (150 mM NaCl, 50 mM Tris-HCI pH 8.0 in water) and transferred into a 1.5 mL reaction tube. Three cycles of freeze/thaw (-80°C for 20 min and 37°C for 5 min) opened the cells and released internal AAV particles. Subsequent benzonase treatment (50 U, 37°C, 1 h; Sigma-Aldrich) reduced residual plasmid DNA contamination. After separation from cell debris (20,000 × g, 4°C, 15 min), aliquots of the resulting crude AAV supernatant was stored at -80°C until use.

For experiments with primary human cells (i.e. CD4+ T cells and PBMC), iodixanol gradient purification of crude AAV supernatant was conducted. After large scale production of crude AAV supernatant (twenty 10 cm dishes, 9 mL lysis buffer, 450 U benzonase), 9 mL of crude AAV supernatant was purified over an iodixanol step gradient as described before. After ultracentrifugation (4°C, 350,000 × g, 90 min; Beckman 70 Ti rotor) the 40% iodixanol fraction containing the AAV2 particles was collected with a needle and syringe. Washing, PBS-Pluronic F68 buffer (Gibco) exchange and further concentration was achieved by filter column centrifugation (Amicon Ultra-15, 100 kDa MWCO; Sigma-Aldrich). Aliquots of purified AAV supernatants were stored at -80°C until use.

Viral titres were determined by qPCR for all AAV supernatants. For this, 1 µL of the AAV supernatant was subjected to DNasel and a subsequent Proteinase K treatment, to eliminate residual plasmid DNA contamination and release viral vector genomes from the capsids. A 100 µL reaction containing 1 µL AAV supernatant (10 U DNasel in 1x DNasel buffer; New England Biolabs) was incubated at 37°C for 16 h followed by DNasel inactivation at 75°C for 30 min. Next, 2 µL of Proteinase K (20mg/mL; QIAGEN) was added and incubated at 55°C for 2 h. Proteinase K was inactivated at 95°C for 30 min. From this reaction, 1 µL was subjected to absolute quantification of vector genomes using a plasmid standard as reference (pscAAV-CAG-GFP). The Platinum^{®} SYBR^{®} Green qPCR SuperMix-UDG kit (Thermo Fisher Scientific) was used according to the manufacturers' instructions in combination with an Applied Biosystem 7500 real-time PCR cycler (qPCR program: initial denaturation 95°C 3 min; 40 cycles of denaturation 95°C 15" and annealing/elongation 60°C 30", and a subsequent melting curve; oligonucleotides oMH93 and oMH94 are listed in Fig. 22).

### Immuno-gold electron microscopy

Iodixanol gradient purified AAV2 samples were adsorbed to 400 mesh carbon coated grids for 10 min. After incubation with blocking solution for protein A/G gold conjugates (Aurion) for 5 min, samples were incubated with primary polyclonal goat anti-alpaca IgG antibody (10 ug/mL, Jackson ImmunoResearch) for 30 min followed by 5 min incubation with blocking solution (see above). Subsequently, secondary antibody rabbit, polyclonal anti-goat IgG 10nm gold-conjugated (1:10 diluted, Sigma) was added for 15 min. After washing with ddH2O five times, samples were stained with 1 % uranyl acetate and air dried. Transmission electron micrographs were taken with a FEI Tecnai G20 equipped with an Olympus Veleta CCD camera at 80kV. Representative images with respective scale bars were cropped for clarity.

### AAV transduction and flow cytometry

If not further specified, cells were transduced with crude AAV2 lysates at the indicated genome copies per cell (gc/cell). One day prior to infection, 2.5×10⁴ cells were seeded per well in a 12-well dish. In mixed culture experiments, HeLa wildtype and HeLa TZMbl cells were seeded in an approximate 1:1 ratio with a total of 2.5×10⁴ cells per well 24 h prior to transduction. For transduction of primary CD4⁺ T cells and PBMC from buffy coats, cultures were pre-stimulated with CD3/CD28 magnetic beads (Invitrogen) for 24 h according to the manufacturer's instructions. After pre-stimulation, cells were counted and seeded in a 96-well plate with 4×10⁴ cells per well. Iodixanol gradient-purified AAV particles were added to the cultures at the indicated genome copies per cell in the presence of 500 U/ml recombinant human IL-2. In all cell culture experiments, cells were harvested three days post transduction, washed with PBS and stained with PE-Cy7 conjugated anti-CD4 antibody (RPA-T4; eBioscience) in PBS with 2% FCS at 4°C for 30 min. After two rounds of washing with PBS, cells were analyzed for CD4 and/or GFP expression. Data was acquired on a Canto II (BD Bioscience) or LSR Fortessa (BD Bioscience) device, analyzed with FlowJo v10.7 software (Tree Star) and presented with GraphPad v9 (Prism).

### Results

### Generation of nanobody-engineered AAVS particles

The respective Nb cDNAs of the above mentioned nanobodies were synthesized and used for genetic modification of AAV2 capsid (cap) genes. Previously, for capsid modification purposes, heterologous sequences were either inserted into the GH2/GH3 surface loop of the common region in VP1, or fused to the amino-terminus of VP2. Using both strategies, selected CD4-Nb-encoding sequences, including Nb tandem constructs, were fused in frame with VP1 and/or VP2 coding sequences, resulting in AAV2 capsid particles displaying human CD4-specific Nbs on their surface (Figure 14). All constructs were generated using self-complimentary AAV2 genome configuration.

In addition, to optimize vector transduction efficiency, four surface-exposed capsid residues were mutated (Y444F, T491V, Y500F, and Y730F) in order to prevent their phosphorylation, a post-translational modification that otherwise may lead to ubiquitination, followed by proteasomal degradation of incoming vector in the target cell's cytoplasm. All vector Nb-modifications were carried out in the cap R585A and R588A background, thereby preventing the resulting vectors from binding primary proteoglycan receptors (blinding). Moreover, all vectors expressed the eGFP marker driven by the constitutive CAG promoter and the polyA site of SV40.

The respective AAV2 CD4-Nb-displaying particles were produced by transient co-transfection of HEK293T cells with AAV helper, capsid and transfer vector plasmids following established protocols (see Materials and Methods above). Selected gradient-purified vector batches were analyzed by electron microscopy, revealing a wildtype-like phenotypic AAV appearance, irrespective of whether the viral particles were Nb-modified (Figure 15A). Furthermore, immune-gold staining using a primary antibody against alpaca IgG revealed accessible nanobody moieties at the capsid surfaces of nanobody-modified vector batched (Figure 15B).

### Transduction of CD4 expressing cell lines

First, various cell lines were analyzed with respect to CD4 surface expression by flow cytometry. As shown, CD4-deficient HeLa wildtype (wt) and CD4+ HeLa TZMbl cells displayed the expected phenotypes, lacking or expressing the CD4 molecule on the cell surface (Figure 16A). Human Jurkat 1G5 T cells expressed CD4 moderately, while the related SupT1 cells were characterized by strong CD4 expression (Figure 16A). Next, these cell lines were transduced individually with AAV vector preparations, comprising the controls AAV2opt (Y444F, T491V, Y500F, Y730F) and AAV2blind (Y444F, T491V, Y500F, Y730F, plus R585A, R588A), as well as a set of capsid (Nb)-engineered vectors, including a negative Nb control (PepNb, recognizing an unspecific control peptide). Transduction of HeLa cells with the control vector AAV2opt resulted in high transduction rates over the entire range of input vector genome copies per cell (gc/cell), ranging in every experiment from 10,000 to 10 gc/cell (Figure 16B upper panel). These results were observed irrespective of whether transduced HeLa cells were negative (HeLa wt) or positive for the CD4 receptor (HeLa TZMbl).

When the control vectors AAV2blind and PepNb were analyzed, comparable low transduction rates could only be recorded at the highest concentration of 10,000 gc/cell, confirming a strong reduction in infectivity due to elimination of primary receptor binding. In contrast, inspection of the various CD4 Nb-specific constructs (CD4-Nb), particularly in the case of CD4-Nb1 and CD4-Nb3 (schematically outlined in Figure 14A), demonstrated maximal transduction in a CD4 receptor-dependent manner at high vector genome copies (Figure 16B, upper panel). Interestingly, CD4-Nb1 displayed a similar transduction efficiency independently of the site of Nb sequence insertion (internal loop in VP1 versus amino-terminus in VP2), while in contrast, CD4-Nb3 only performed well when VP1 was genetically modified.

Similar results were obtained in the T cell lines SupT1 and Jurkat 1G5 (Figure 16B, lower panel).Although transduction efficiencies were overall lower across the entire range of input vector (i.e. gc/cell), CD4-Nb1 and CD4-Nb3 demonstrated comparable and maximal transduction rates of SupT1 cells in particular, which stained highly positive for CD4 (Figure 16A). As seen above, the CD4-Nb3 construct only positively affected transduction of CD4+ cells when expressed as a fusion in VP1. These data were also confirmed using Jurkat 1G5 cells transduced with the CD4-Nb1 or CD4-Nb3 vectors using 10,000 gc/cell and double-stained for eGFP and CD4 followed by flow cytometry analysis at day 3 post transduction (Figure 16C). Inspection of the flow cytometry plots revealed enhanced transduction of cells strongly expressing CD4. As above, this effect was observed for CD4-Nb1 VP1- and VP2-modified viral particles, as well as for CD4-Nb3 VP1-modified AAV2.

Taken together, these experiments indicated that CD4-Nb1 and CD4-Nb3 are useful reagents for targeting AAV2-derived gene vectors to cells, particularly those expressing CD4 on the surface.

### Preferred targeting of CD4+ cells in mixed cell cultures

Next, CD4-specific cell targeting by AAV2 CD4-Nb1 and CD4-Nb3 constructs was analyzed in competition experiments, where two distinct cell populations, CD4-negative HeLa wt cells and CD4-positive HeLa TZMbl cells, were mixed in a ratio of 1:1. In agreement with the results above, addition of the respective CD4-Nb-modified vector particles and control vectors to these mixed cell cultures, followed by flow cytometry, clearly revealed preferred transduction of the CD4-expressing subpopulation in the respective cultures (Figure 17A). In contrast, the AAV2opt vector again efficiently and promiscuously transduced target cells at all vector input concentrations and irrespective of whether the CD4 surface receptor was expressed (compare Figure 16B and 17A). The remaining vector controls, AAV2blind and PepNb, also showed indiscriminate cell transduction, however as recorded before (Figure 16B), only at the highest vector particle concentration used (10,000 gc/cell).

Subsequently, the relative frequencies of eGFP-specific signals in CD4+ and CD4- cells from three full sets of independent experiments were calculated and presented as CD4+/CD4- ratios (Figure 17B). These data confirmed the expected CD4-Nb-dependent increase in transduction efficiencies, especially when the Nb1- and Nb3-encoding sequences were fused to VP1, and moderate or low input vector particles (i.e. 1,000 to 10 gc/cell) were used. The respective frequencies indicated a 20- to 199-fold increase in the transduction rate of CD4+ over CD4- tissue culture cells (Figure 17B).

It has been previously reported that under certain conditions homologous Nb tandem-repeats may increase the affinity of a specific Nb. Therefore, CD4-Nb1 was inserted into VP1 as a homodimer (depicted in Figure 14B) and tested as described above. The analyses revealed that bivalent CD4-Nb1 (i.e. a tandem Nb1 repeat), although displaying specificity comparable to the monovalent Nb1, is not more potent in infecting specifically CD4+ cells (Figure 19). This may be explained by some structural interference, since construction of the bivalent Nb1 vector required an intragenic insertion of 885 nucleotides (nt), a size that is clearly larger than the maximal heterologous insertion reported for this site before, which comprised a 720 nt sequence encoding the mCherry marker protein.

In summary, these data indicate that CD4-Nb-modified AAV2 vectors allow specific targeting of CD4 receptor-expressing cells in mixed cell cultures. Moreover, monovalent Nb1 capsids already appears to show specificity and high transduction efficiencies, even at relatively low vector doses.

### Enhanced transduction of primary human CD4+ cells

Since AAV-derived gene therapy vectors are primarily used for in vivo delivery of a heterologous genetic payload, transduction of primary human CD4+ T cells as well as human PBMC were analyzed next. First, purified CD4+ T cells from two healthy donors were transduced and assayed with respect to GFP-positive cells as above. Here, a mock transduction control, the vector controls AAV2opt and PepNB, was included as well as an AAV6 construct. The latter were included due to the fact that AAV6 vectors have been reported to show tropism for multiple tissues, and are able to transduce various human primary cells, including dendritic cells (DC), CD34+ hematopoietic stem and progenitor cells (HSC), and CD8+ and CD4+ T cells. As opposed to the human cell lines investigated above, the transduction efficiencies were clearly lower in primary cells (Figure 18A). Nonetheless, successful transduction of CD4+ T lymphocytes was observed, particularly by AAV2 CD4-Nb1 and AAV6. To elucidate transduction specificities, a human PBMC culture was then transduced and ratios of transduced CD4+/CD4- cells as outlined above were calculated. AAV2 CD4-Nb1- and Nb3-modified vector particles transduced CD4+ cells about 4- to 5-fold more efficiently than cells lacking or only weakly expressing the CD4 receptor (Figure 18B). As expected, due to its rather broad tropism, AAV6 failed to specifically transduce CD4+ PBMC in this analysis.

Taken together, these data demonstrate that CD4 Nb capsid-engineered AAV2 vectors show an improved tropism for CD4+ human PBMC.

### Summarizing Discussion

Within the present invention, AAV vector tropism could be successfully altered and thereby its clinical applicability by specific capsid engineering to achieve enhanced targeting of CD4+ cells by appropriately capsid-modified AAV2 vectors.

In the present study, AAV2 capsids were modified using the above mentioned nanobodies of the invention. The presentation of these Nbs at the surface of the capsid VP1 protein appeared to be more effective as an amino-terminal VP2 fusion (compare Figure 16 and Figure 17). Likewise, the identical insertion of a homologous tandem repeat of CD4-Nb1 into VP1 also resulted in enhanced, but not further improved, specificity of this vector for CD4+ cells (Figure 19). These findings confirmed that this site in VP1 can indeed accommodate at least two Nb-encoding sequences, potentially allowing the insertion of heterologous Nb tandem repeats to target different receptors using the same AAV vector construct.

Clearly, the Nb-mediated capsid modifications presented here improved the ability of AAV2 vectors to target CD4+ cells, including primary human T lymphocytes. The latter are of particular interest for developing novel experimental therapies, for instance in the field of therapeutic genome editing, where direct, specific and efficient introduction of designer nucleases or recombinases into CD4+ lymphocytes is required. For example, the chromosomally integrated genome of human immunodeficiency virus (HIV; i.e. the provirus) persists indefinitely within CD4+ T cells and cannot be eradicated by classical antiretroviral therapy. However, potential direct in vivo delivery of such an antiviral designer recombinase using CD4-Nb-targeted AAV gene vectors may ultimately enable the clinical development of a scalable curative therapy for HIV/AIDS.

In conclusion, the findings presented herein represent an important step towards the development of more potent AAV vectors for specific gene transfer into human CD4+ T lymphocytes.

In summary, the present invention and the data presented in connection therewith, on the one hand, demonstrate for the first time the generation and detailed characterization of Nbs specific for human CD4 and their application in various biomedical research approaches. In particular, CD4-Nb1 is a promising candidate for the noninvasive, whole-body study of CD4+ T cells in mice, as well as in humans.

## Claims

1. A nanobody which binds to human CD4, the nanobody comprising
a) the amino acid sequences (i) SGFTFSKL (SEQ ID NO: 1) as CDR1, (ii) IDSSGDTTDYLA (SEQ ID NO: 2) as CDR2 and (iii) REDPPG (SEQ ID NO: 3) as CDR3; or
b) the amino acid sequences (i) SGFDVDYY (SEQ ID NO: 4) as CDR1, (ii) IASSDGSTYYAD (SEQ ID NO: 5) as CDR2 and (iii) DATCPYYCSGSVCYLETGMD (SEQ ID NO: 6) as CDR3; or
c) the amino acid sequences (i) SGFALEYY (SEQ ID NO: 7) as CDR1, (ii) MSASGGVINYSE (SEQ ID NO: 8) as CDR2 and (iii) EKAYYGSSWAECYLMMD (SEQ ID NO: 9) as CDR3; or
d) amino acid sequences that have at least 90% sequence homology with the amino acid sequences as defined in a), b) or c),
or a functionally conservative variant of the nanobody as defined in any of a), b) or c) comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9;

2. The nanobody of claim 1, wherein the nanobody binds to domain D1 or to domain D3 of human CD4.

3. The nanobody of claim 1 or 2, comprising four framework regions (FR1 to FR4) and three complementarity determining regions (CDR1 to CDR3), the three complementarity determining regions consisting of
(i) one of the amino acid sequences a) to c), or of
(ii) an amino acid sequence that has at least 90% sequence homology with one of the amino acid sequences a) to c), or of
(iii) an amino acid sequence comprising a conservative substitution of one or two amino acids in one, two or three of the sequences, respectively, SEQ ID No. 1, SEQ ID No. 2, and SEQ ID No. 3; or SEQ ID No. 4, SEQ ID No. 5, and SEQ ID No. 6; or SEQ ID No. 7, SEQ ID No. 8, and SEQ ID No. 9;
wherein preferably, the nanobody comprises an amino acid sequence selected from the group consisting of the amino acid sequences SEQ ID NO. 10, SEQ ID NO. 11, SEQ ID NO. 12, , or an amino acid sequence that has at least 90% sequence homology with one of the amino acid sequences SEQ ID NO. 10, SEQ ID NO. 11, SEQ ID NO. 12,

4. The nanobody of any of claims 1 to 3, wherein the nanobody is associated with at least one of a detectable label, a viral particle, and/or a therapeutically or pharmacologically active agent.

5. The nanobody of claim 4, wherein the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI), and preferably which detectable label is selected from fluorophores, radionuklids or magnetic particles.

6. The nanobody of claim 4, wherein the viral particle is an Adenovirus-associated Virus (AAV) particle, an Adenovirus particle, or an lentiviral particle, and wherein preferably the AAV particle is selected from an AAV2, AAV3, AAV4, AAV5, AAV6, AAV7, AAV8, or an AAV9 particle.

7. The nanobody of claim 4, wherein the therapeutically active agent is an active gene transfer agent.

8. A nucleic acid comprising or consisting of a nucleic acid sequence coding for the nanobody as claimed in any of claims 1 to 7, optionally linked to another nucleic sequence.

9. The nanobody of any of claims 1 to 6, or the nucleic acid of claim 8, for use in diagnostic or prognostic methods, or for use as medicament, preferably for modulating CD4⁺ T cell function and/or cell depletion.

10. The nanobody of any of claims 1 to 7 for use as contrast agent in non-invasive medical imaging *in vivo.*

11. The nanobody of any of claims 1 to 7 for the *in vitro* detection of CD4 in a sample.

12. The nanobody of any of claims 1 to 7, or the nucleic acid as claimed in claim 8, for use in retargeting AAV, Adenovirus or lentiviral vectors or for retargeting gene transfer vectors, or for use in targeting virus-like particles (VLP) or lipid nanoparticles (LNP).

13. The nanobody of any of claims 1 to 7, or the nucleic acid as claimed in claim 8, wherein the nanobody or a nucleic acid coding for the nanobody is used for genetic labelling of AAV capsid proteins, preferably for genetic labeling of the AAC capsid proteins VP1 and/or VP2.

14. A biparatopic, bivalent or trivalent construct comprising at least one nanobody as claimed in any of claims 1 to 7 or a combination thereof.

15. A method for determining and/or imaging and/or monitoring the presence and/or amount and/or activity or differentiation state of CD4⁺ cells in a biological sample suspected of comprising CD4⁺ cells, preferably CD4⁺ T cells, the method comprising the steps of:
a) combining said sample with at least one of the nanobodies of any of claims 1 to 7, or with at least one construct of claim 14, and optionally with at least one second compound conjugated with a detectable label, the second compound binding to another marker of the CD4+ cells, preferably CD4⁺ T cells,
b) measuring, via the detectable label, imaging signals from said nanobody that has bound to CD4⁺ cells, preferably CD4⁺ T cells, in said sample, and, if a second compound has additionally been used in step a), from said second compound that has bound to another marker; and
c) imaging, determining and/or monitoring the presence and/or amount and/or activity of CD4⁺ cells, preferably CD4⁺ T cells, in said sample via the imaging signals,
wherein the determination and/or monitoring and/or imaging is preferably for patient stratification and for monitoring individual immune responses during personalized immunotherapy.

16. Use of at least one of the nanobodies of any of claims 1 to 7, or at least one construct of claim 14, as a diagnostic agent, preferably wherein the diagnostic agent is used in a diagnosis which is selected from diagnostic classification of a disease-associated immune status of a patient, and susceptibility to immunotherapies of a patient.

17. A method for diagnosing and/or monitoring the immune status and/or susceptibility to immunotherapies of a patient, wherein at least one nanobody as claimed in any of claims 1 to 7, or at least one construct as claimed in claim 14, is used to assess and/or monitor CD4⁺ T cells in a biological sample of the patient, wherein the nanobody is used alone or in combination with a second compound binding to a T-cell specific marker, wherein the T cell-specific marker is selected from at least one of CD2, CD3, CD7, CD8, CD27, CD28, CD127, HLA-DR, CD38, CD69, CCR4, CCR5, CCR6, CCR7, CTLA-4, LAG3, TIM-3, OX40, ICOS, CXCR4, PD-1, PD-L1, PD-L2, CD40L (synonym CD154), CD122, CD137, GITR, CD25, CD278, SIGLEC-7, SIGLEC-9, BTLA (synonym: CD272), TIGIT, VISTA, B7-H4 (synonym: VTCN1), CD276 (synonym: B7-H3), A2AR, CEACAM1, LAIR-3, HVEM, CD160, CD200, CD200R.

18. A pharmaceutical composition comprising at least one of the nanobodies as claimed in any of claims 1 to 7 and/or at least one construct as claimed in claim 14, in association with a pharmaceutically acceptable carrier or excipient.
